# EUROPÄISCHE PATENTSCHRIFT

(11) **EP 4 340 939 B1**
(45) Veröffentlichungstag und Bekanntmachung des Hinweises auf die Patenterteilung: **01.07.2026**
(21) Anmeldenummer: 22730599.2
(22) Anmeldetag: 03.05.2022
(51) Int. Cl.: A61N 5/10

(54) **MCO AUSWAHL VON BEHANDLUNGSTECHNOLOGIEN BEI DER RADIOTHERAPIE (VERFAHREN, TECHNISCH FUNKTIONALE GUI UND VERWENDUNG)**
MCO SELECTION OF TREATMENT TECHNOLOGIES IN RADIOTHERAPY (METHOD, TECHNICALLY FUNCTIONAL GUI, AND USE)
SÉLECTION MCO DE TECHNOLOGIES DE TRAITEMENT EN RADIOTHÉRAPIE (PROCÉDÉ, INTERFACE GRAPHIQUE UTILISATEUR TECHNIQUEMENT FONCTIONNELLE ET UTILISATION)

(30) Priorität: 03.05.2021 DE 102021111420; 03.09.2021 DE 102021122848
(43) Veröffentlichungstag der Anmeldung: 27.03.2024
(73) Patentinhaber: Fraunhofer-Gesellschaft zur Förderung der angewandten Forschung e.V., 80686 München (DE)
(72) Erfinder: BORTZ, Michael, 67663 Kaiserslautern (DE); KUEFER, Karl-Heinz, 67685 Weilerbach (DE); TEICHERT, Katrin, 67655 Kaiserslautern (DE); SUESS, Philipp, 55257 Budenheim (DE)
(74) Vertreter: Leonhard, Frank Reimund
(86) Internationale Anmeldenummer: PCT/IB2022/054081
(87) Internationale Veröffentlichungsnummer: WO 2022/234453

(56) Entgegenhaltungen:
- EP-A1- 3 669 939
- US-A1- 2013 090 549

## Beschreibung

Diese Offenbarung (und Ansprüche) betreffen ein Verfahren der multikriteriellen Optimierung (MCO). Auch betroffen ist eine GUI, eigenständig, oder zur Anwendung (Verwendung) in diesen Verfahren. So betrachtet ist die Verwendung eine neue Verwendung eines als solchen bekannten Funktionszeigers.

Ein Krebspatient soll eine Strahlentherapie erhalten. Hierfür ist eine Mindestdosis für das Tumorgewebe vorgesehen und Höchstdosen für umliegende gesunde Gewebestrukturen, die idealerweise nicht ausgeschöpft werden sollten. Prinzipiell kommen für einen solchen Patienten mehrere technische Möglichkeiten (Technologien) in Frage, mit denen eine solche Behandlung durchgeführt werden kann. Beispielhaft zu nennen sind:
1. Protonen
2. Photonen per Rotationstherapie mit 1 Umdrehung
3. Photonen per Rotationstherapie mit 2 Umdrehungen
4. Photonen per IMRT mit 9 Feldern (mit 9 festgesetzten Winkeln)
5. Photonen per IMRT mit 9 Feldern (mit anderen Winkeln als in Option 4)
6. Weitere Technologien.

Jede dieser Technologien kann auf einem oder mehreren verschiedenen Therapiegeräten durchgeführt werden. Jedes Gerät ist anders anzusprechen und bedarf einer spezifischen Planung, wobei die individuellen Planziele über die eingesetzten Technologien identisch bleiben.

Aus Sicht der Klinik relevant ist auch das Planen aller Behandlungsfälle (Gesamtplanung) unter Berücksichtigung aller zur Verfügung stehender Behandlungsgeräte. Die Gesamtplanung soll nicht nur unter den individuellen medizinischen Aspekten eine Therapie gerecht werden, sondern gleichzeitig eine Terminierung der Behandlungen vorgenommen werden.

Mathematisch entsteht ein mehrkriterielles Planungsproblem mit den Zielen: hohe Wahrscheinlichkeit eines Therapieerfolgs bei gleichzeitiger Vermeidung von Nebenwirkungen bei jeder individuellen Therapie und möglichst effektive Nutzung der zur Verfügung stehenden Therapiegeräte, mit denen möglichst viele Patienten eine Therapie erhalten können, ohne lange Wartezeiten in Kauf nehmen zu müssen.

Diese Offenbarung (und Ansprüche) betreffen ein Verfahren der multikriteriellen Optimierung (MCO). Auch betroffen ist eine GUI, eigenständig, oder zur Anwendung (Verwendung) in diesen Verfahren. So betrachtet ist die Verwendung eine neue Verwendung eines als solchen bekannten Funktionszeigers.

Ein Krebspatient soll eine Strahlentherapie erhalten. Hierfür ist eine Mindestdosis für das Tumorgewebe vorgesehen und Höchstdosen für umliegende gesunde Gewebestrukturen, die idealerweise nicht ausgeschöpft werden sollten. Prinzipiell kommen für einen solchen Patienten mehrere technische Möglichkeiten (Technologien) in Frage, mit denen eine solche Behandlung durchgeführt werden kann. Beispielhaft zu nennen sind:
1. Protonen
2. Photonen per Rotationstherapie mit 1 Umdrehung
3. Photonen per Rotationstherapie mit 2 Umdrehungen
4. Photonen per IMRT mit 9 Feldern (mit 9 festgesetzten Winkeln)
5. Photonen per IMRT mit 9 Feldern (mit anderen Winkeln als in Option 4)
6. Weitere Technologien.

Jede dieser Technologien kann auf einem oder mehreren verschiedenen Therapiegeräten durchgeführt werden. Jedes Gerät ist anders anzusprechen und bedarf einer spezifischen Planung, wobei die individuellen Planziele über die eingesetzten Technologien identisch bleiben.

Aus Sicht der Klinik relevant ist auch das Planen aller Behandlungsfälle (Gesamtplanung) unter Berücksichtigung aller zur Verfügung stehender Behandlungsgeräte. Die Gesamtplanung soll nicht nur unter den individuellen medizinischen Aspekten eine Therapie gerecht werden, sondern gleichzeitig eine Terminierung der Behandlungen vorgenommen werden.

Mathematisch entsteht ein mehrkriterielles Planungsproblem mit den Zielen: hohe Wahrscheinlichkeit eines Therapieerfolgs bei gleichzeitiger Vermeidung von Nebenwirkungen bei jeder individuellen Therapie und möglichst effektive Nutzung der zur Verfügung stehenden Therapiegeräte, mit denen möglichst viele Patienten eine Therapie erhalten können, ohne lange Wartezeiten in Kauf nehmen zu müssen.

Ein Stand der Technik ist ein zweistufiger Entscheidungsansatz. Zuerst wird die Technologie festgelegt, dann wird diese Technologie geplant. Dabei können beim zweiten Schritt mehrkriterielle Planungsmethoden zum Einsatz kommen. Die Entscheidung über die Auswahl der Technologie treffen Ärzte aufgrund von Erfahrung und Ergebnissen klinischer Studien. Will man entscheiden, welche Technologie sich für einen Behandlungsfall am besten eignet, muss für jede mögliche Technologie eine separate Planung durchgeführt werden, die jeweiligen Pläne verglichen und dann die "beste" ausgesucht werden.

Ein anderer Stand der Technik ist US 2013/090549, der im Oberbegriff abgebildet ist.

Der klinische Ablauf ist auch zu bedenken: für dringende Fälle müssen bereits auf bestimmte Geräte terminierte Behandlungen verschoben oder sogar auf andere Geräte umgeplant werden. Oder der dringend eine Therapie benötigende Patient muss warten.

Jede Technologie muss mit bestimmten, dafür vorgesehenen Geräten erfolgen - eine Behandlung mit Protonen kann nicht mit einem Photonenbeschleuniger erfolgen. Die Anzahl der zur Behandlung verfügbaren Geräte ist einer der limitierenden Faktoren für Krankenhäuser. Mehr Patienten zu behandeln - gerade Protonenanlagen sind auf Grund ihrer Kosten und Komplexität nur sehr begrenzt verfügbar - zwingt zu Wartezeiten oder Therapie-Umplanungen. Erkennbar wird ein mehrkriterielles Optimierungsproblem; möglichst viele Patienten in gegebener Zeit - oder bei gegebenem, meist beschränktem Investitionskapital, behandeln zu können.

**Das technische Problem der Erfindung** liegt darin, jedem Patienten individuell eine gute Therapie zukommen lassen zu können, wobei die Güte der Therapie auch die Minimierung der Wartezeit jedes Patienten einbezieht (unter "time to treatment" ein technisches Kriterium einer Therapieplanung).

Die Erfindung geht von der Erkenntnis aus, dass für einzelne Patienten aufgrund von weiter unten beschriebenen Unsicherheiten eine Protonenbehandlung als nicht eindeutig besser identifiziert werden kann, der Behandlungsfall auch mit einer Variante der Photonentherapie gelöst werden kann. Damit lassen sich Strahlengeräte für die Protonenbehandlung freihalten für Fälle wo diese unabdingbar sind, oder Behandlungen können bei ein wenig mehr Zeitaufwand auf z.B. einem Photonen-Strahlengerät ausgeführt werden.

Die Navigation einer Paretofront über Patches hinweg (ein Patch entspricht einer Technologie) wird ergänzt durch einen Unschärfebereich, wobei ein Schwellenwert angeben kann, so dass unterhalb seines Werts von z.B. 10% zwei Technologien zwar nicht gleich, sondern gleichwertig sind, also beide gleichermaßen für die Therapie verwendet werden könnten.

umschrieben, dass sie zumindest zwei Paretofronten (die für zumindest zwei Technologien stehen) erfasst. Durch eine Eingabe des Planers wird mit der Unschärfe eine Gleichwertigkeit von zwei Technologien visuell vermittelt, wobei die beiden dargestellten Technologien einen gleichen Wert c1₁ für das gleiche Zielkriterium (z.B. c1) in allen Patches aufweisen, die Unschärfe aber nicht so weit reicht, dass nicht gleichwertige Technologien erreicht werden.

Ein anderes im Bedienbereich dargestelltes Zielkriterium hat die zum angenommenen Wert c1₁ keine gleichen Werte.

Auf dem Sichtgerät kann - muss aber nicht - eine resultierende Paretofront als Patch dargestellt werden, die mehrere Abschnitte von mehreren bereits dargestellten Paretofronten als Patches dargestellter Technologien nachführt (oder aufgreift), um es dem Planer sichtbarer oder deutlicher zu machen, dass er technologie-übergreifend auf dem resultierenden Patch (801) navigiert, für ein Auffinden eines Behandlungsplans. Die resultierende Paretofront kann eine nichtdominierte Menge einer Vereinigung der Paretofronten sein.

Aus zwei dargestellten Patches kann ein kombinatorisches Patch mit ersten Anteilen des ersten Patches und zweiten Anteilen des zweiten Patches berechnet werden und zur Navigation des Planers auf dem Sichtgerät dargestellt werden. Es entsteht ein weiteres Patch auf dem Sichtgerät, das sich aus Anteilen der dargestellten Patches ableitet. Auf ihm kann der Planer ebenso navigieren, wie auf den Patches, welche die Technologien abbilden, oder der resultierende Paretofront. Letztere muss nicht dargestellt sein.

Ein alternatives Verfahren erlaubt das Entwerfen oder Gestalten eines Behandlungsplans mit einer interaktiven Navigation an einem Eingabe-/Ausgabe Gerät. Das Gerät umfasst ein Sichtgerät. Auf dem Sichtgerät wird für zumindest einige der Technologien je eine Paretofront in einem Paretofront-Diagramm dargestellt. Jede der Paretofronten soll Patch genannt werden. Jede der Paretofronten wird in Abhängigkeit von zumindest zwei Kriterien in dem Patchbereich des Sichtgeräts dargestellt. In einem Bedienbereich, außerhalb des Patchbereichs, wird eine Anzahl von Bedienhilfen dargestellt. Diese können als Slider dargestellt sein.

Einem Planer stehen mehrere Behandlungstechnologien für seinen zu entwerfenden oder zu gestaltenden Plan zur Auswahl. Der entworfene oder gestaltete Behandlungsplan definiert eine Vielzahl von technischen Einstellungen, welche an dem zumindest einen Strahlengerät der ausgewählten Technologie(n) einstellbar sind.

Für zumindest einige der Technologien wird je eine Paretofront in dem Paretofront-Diagramm dargestellt. Jede der Paretofronten wird in Abhängigkeit von zumindest zwei Kriterien in dem Patchbereich dargestellt. Die Kriterien werden von Bedienhilfen dargestellt.

Jede Bedienhilfe ist einem Kriterium zugeordnet, oder anders herum. Das Ändern eines Kriteriums erfolgt mit einem Selektor. Bei einem Bewegen oder Bedienen eines Selektors der jeweiligen Bedienhilfe in eine Richtung wird das Kriterium verbessert oder in eine gegenläufige Richtung wird das Kriterium verschlechtert.

Jede Bedienhilfe stellt mit Hilfe eines Selektors - für eine interaktive Kommunikation mit dem Planer - eine im aktuellen Zustand ausgewählte Lösung in dem Patchbereich als Punkt zᵢ auf einer der mehreren Paretofronten dar. Die ausgewählte Lösung wird bei der interaktiven Kommunikation mit dem Planer entlang mehrerer Patches verändert.

Es soll ein Unschärfeintervall, das bei einem Zielkriterium definiert wird, vorgesehen sein. Es wird auch im interaktiven Patchbereich entsprechend repräsentiert angezeigt und erfasst zumindest zwei Paretofronten (als zumindest zwei Behandlungstechnologien). Dadurch wird interaktiv durch eine Eingabe des Planers eine Gleichwertigkeit von zwei Technologien demselben Planer vom Patchbereich vermittelt. Die beiden dargestellten Behandlungstechnologien weisen einen gleichen Wert c1₁ für das gleiche Zielkriterium c1 im jeweiligen Patch auf.

Die Lage des Selektors einer Bedienhilfe linear (als Slider) verändert werden, oder als Drehlage verändert werden (rotatorische Vorgabe). Die Bedienhilfen sind dabei auf dem Sichtgerät abgebildet und können per Mauszeiger oder an einem touch-enabled Screen (Touchscreen) direkt mit der Hand eines Fingers bedient werden.

Beim Slider ist die Bedienhilfe linear ausgebildet. Bei einer Bedienhilfe mit veränderbarer Drehlage hat die die Bedienhilfe einen gebogenen Verstellbereich. In der Regel werden die Bedienhilfen zweidimensional abgebildet, was sie in ihrem Verständnis indes nicht beschränkt.

Es können mehr als zwei Zielkriterien (auch Planziele) durch Projektionen im Patchbereich auf dem Sichtgerät dargestellt werden. Die Abbildung von zwei Achsen ist eine starke Vereinfachung, die durchaus in mehrere Dimensionen erstreckt werden kann. Eine Art der Darstellung ist die Projektion.

Eines der Zielkriterien kann eine Behandlungszeit sein. Ein anderes Zielkriterium kann die Zahl der Fraktionen (einer Behandlung) sein. Weitere Zielkriterien sind möglich, das angegebene Kriterium ist kein solitäres Kriterium.

Das Unschärfeintervall kann durch Verbreitern des Selektors der Bedienhilfe interaktiv dargestellt werden.

Es kann für die interaktive Kommunikation mit dem Planer (dem Benutzer des Tools) auch im Patchbereich durch ein erweitertes Feld als Fenster repräsentiert werden. Es erfasst dabei zumindest zwei Paretofronten als zumindest zwei Patches.

Auf dem Sichtgerät kann das Fenster angezeigt werden, in welchem der Planer ausgewählte Technologien (auch Behandlungstechnologien) kennzeichnet, also auswählt oder abwählt. Das Fenster kann so ausgebildet sein, dass es vom Benutzer per Mauszeiger oder per Touchscreen bedient werden kann, um darin angezeigte Technologien (oder Behandlungstechnologien) zu markieren.

Das Fenster wird im Bedienbereich zu einem Funktionsfeld. Die mit ihm (oder in ihm) dargestellten Technologien (oder Behandlungstechnologien) entsprechen den Paretofronten. Z.B. zur Auswahl oder Abwahl durch den Planer. Bei Auswahl werden diese in den Bedienhilfen mit angezeigt oder dargestellt. Ist die Technologie nicht angewählt (oder ausgewählt), werden diese als Bedienelement in der zugehörigen Bedienhilfe nicht angezeigt. Die Auswahl oder Abwahl kann zeitlich mehrfach nacheinander erfolgen.

Eine ausgewählte Lösung kann dadurch entlang des zumindest einen Patches verändert werden, dass der Planer einen jeweiligen Selektor einer jeweiligen Bedienhilfe in seiner Lage verändert. Es kann mehrere Arten der Veränderung der Lage eine Veränderung der Lage des Selektors geben, z.B. durch Verändern der Lage des Punkts im Patchbereich; oder durch Verändern der Lage des Selektors einer jeweiligen Bedienhilfe im Bedienbereich.

Der Patchbereich kann so auch ein Bedienbereich werden, anders gewendet, aus dem Patchbereich kann auch bedient werden.

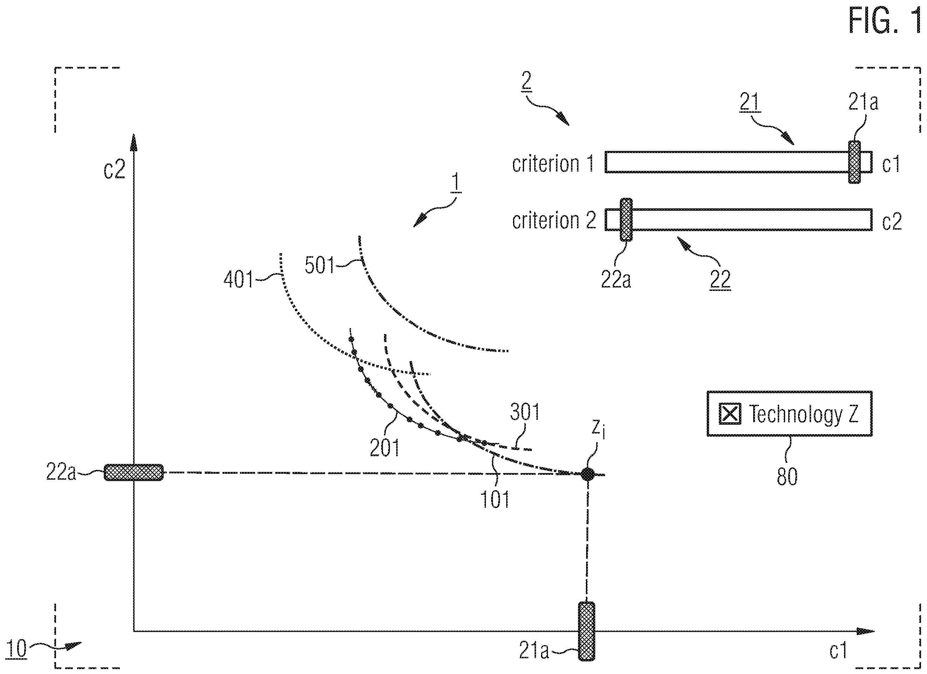

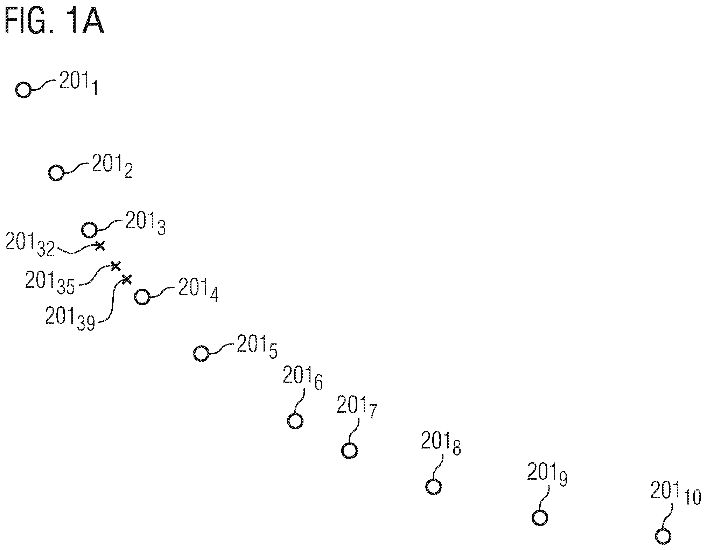

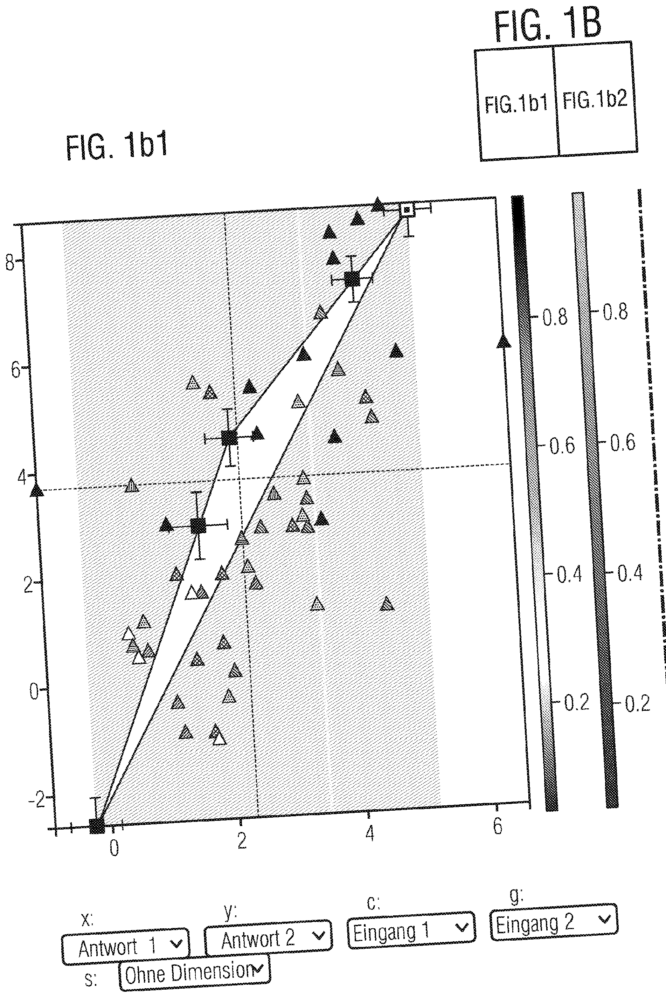

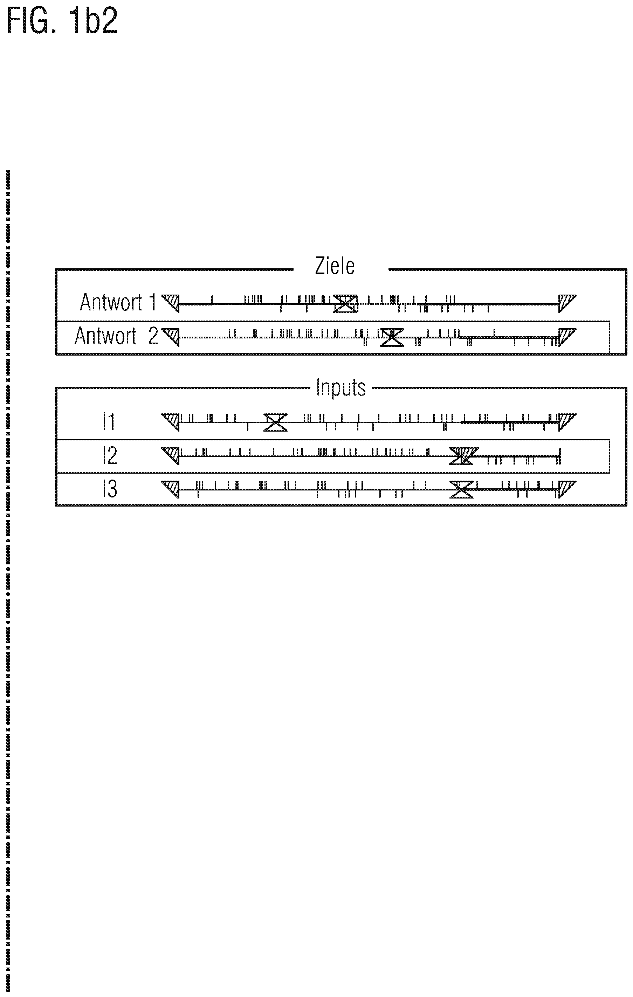

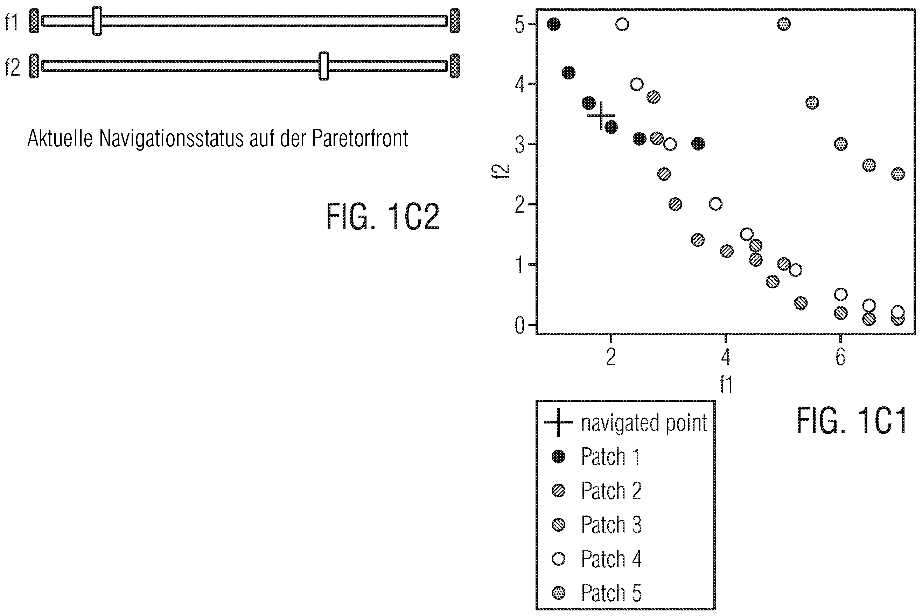

Zum Verständnis der Paretofront(en) soll folgendes angemerkt werden. Jede Paretofront ist eine nur funktionell zusammenhängende Funktion. Jede davon hat ein Vielzahl von Stützstellen. Die Front ist also keineswegs als durchgehend zu verstehen, sondern nur "funktional zusammenhängend", daher ist in der Figur 1 zu den Stützstellen auch eine Linie ausgewiesen, die im Grunde keine durchgehende Paretofront darstellt. Sie kann ebenso eine Projektion aus dem n-dimensionalen Raum sein, in welchem Raum sie besteht, indes grafisch nur als eine Projektion dargestellt werden kann. Die Linien repräsentieren die jeweils funktionell zusammenhängende Paretofront. Mit Bezug auf Figur 1A wird das vergrößert verdeutlicht, zwischen den Stützstellen wird interpoliert.

Zum Verständnis der allgemeinen Aussagen helfen hier die Figuren als Beispiele.

Erreichbare Punkte auf den mehrdimensionalen Paretofronten können optisch anders dargestellt werden. Im Fadenkreuz z.B. der Figur 1b1 ist die aktuelle Stellung (der navigierte Punkt). Erreichbare Punkte können hell oder kontrastierend dargestellt werden. Nicht erreichbare Punkte, z.B. eine projizierte Fläche haben einen anderen Kontrast oder eine andere Farbe.

Mit nicht dargestellter Farbkennzeichnung kann eine nicht sichtbare, indes vorhandene Dimension repräsentiert werden. In fünf größeren Quadraten können dazu Unsicherheiten repräsentiert sein. Eine Mitte davon wäre jeweils der Erwartungswert, jeweils vier Arme in jeweils 90° Orientierungen zeigen jeweils als Intervall solche Bereiche der Unsicherheit an.

Als Strahlengerät können interne oder externe Strahlengeräte verwendet werden. Das zumindest eine Strahlengerät umfasst zumindest ein Strahlengerät, zur Abgabe von Protonen oder Photonen, zur Abgabe von beschleunigten schweren Ionen, zur Abgaben von beschleunigten Elektronen, oder ist ein Brachygerät zur Abgabe von Photonen oder Gammastrahlen aus (individuell) vorgeschobenen (radioaktivem Zerfall unterliegenden) Strahlungskörperchen. Diese Strahlungskörperchen können eine zylindrische Ausbildung haben, die eine Länge von weniger als 5mm haben und aus Cobalt-60, Jod-125 oder Iridium-192 gebildet sind, bevorzugt umgeben von einem Titanmantel. Andere radioaktiv zerfallende Stoffe sind ebenso möglich.

Unter einem beanspruchten Strahlengerät soll also auch ein Gerät der Brachytherapie verstanden werden, z.B. ein Afterloader. Beim Nachladeverfahren (sog. Afterloading) wird die Strahlenquelle computergesteuert von einer Art Roboter eingesetzt. Der Patient liegt dabei allein in einem strahlendichten Raum. Das medizinische Personal kommt so nicht direkt mit den Strahlenquellen in Kontakt, die zuvor als "Strahlungskörperchen" angegeben waren. Dies aufgrund ihrer Größe, nicht aufgrund ihrer Unbedenklichkeit.

Sinngemäß gibt das Strahlengerät also von extern Strahlung zum Patient ab (Radiotherapie), oder von intern (bei eingelegtem radioaktiven, also Photonen oder Gamma Strahlung abgebenden Strahlungskörperchen).

Brachytherapie wird zur Behandlung von Gebärmutterhals-, Prostata-, Brust- und Hautkrebs eingesetzt, und das als LDR (Low-Dose-Rate-Brachytherapie) von bis zu 2 Gy/h (Gray/h), MDR (Medium-Dose-Rate-Brachytherapie) mit einer mittleren Dosisleistung zwischen 2 bis 12 Gy/h), als HDR (High-Dose-Rate-Brachytherapie) mit einer Dosisleistung oberhalb von 12 Gy/h oder als PDR (Pulsed-Dose-Rate-Brachytherapie), bei der kurze Strahlungsimpulse abgegeben werden, z.B. einmal in der Stunde, um als Gesamtintensität und Effektivität einer LDR-Behandlung nachzuahmen.

Bei allen Varianten ist die Planung beansprucht, nicht die eigentliche Behandlung.

Bei der Brachytherapie erfolgt das Einbringen der Strahlenquelle in den (menschlichen) Körper in mehreren Schritten. Zuerst werden ein oder mehrere Applikatoren eingesetzt. Diese Hilfsmittel für das Einbringen einer oder mehrerer Strahlenquellen (den Strahlungskörperchen) können zum Beispiel dünne Röhrchen (Katheter oder Kanülen) aus Metall oder Kunststoff sein. Mithilfe von Röntgenaufnahmen, Ultraschall, Computertomografie oder Magnetresonanztomografie (Kernspintomografie) wird geprüft, ob die Applikatoren an der richtigen Stelle im Körper liegen. Wenn dies der Fall ist, wird in einem weiteren Schritt die Strahlenquelle(n) eingeführt.

Bei der Brachytherapie liegt die Strahlenquelle also entweder in unmittelbarer Nähe zum Tumor (dem Target) oder sie wird direkt in den das Target eingebracht. Dabei legen die Strahlen im Körper nur einen kurzen Weg zurück (altgriechisch "brachys" heißt "kurz", "Nähe", woraus sich der Name dieser Therapie ableitet).

Eine Strahlentherapie bösartiger Targets hat das Ziel, den Tumor zu zerstören, das benachbarte gesunde Gewebe dabei aber möglichst zu schonen. Letzteres ist ein besonderes Anliegen der Brachytherapie, bei der radioaktive Strahler in einem kleinen Eingriff direkt in das Target oder seine unmittelbare Umgebung eingesetzt werden. Anders als bei einer Bestrahlung von außen erreichen die Strahlen das Target direkt und schädigen so weniger das umliegende gesunde Gewebe.

Historisch ging nach einem anfänglichen Interesse an der Brachytherapie in Europa und den USA ihre Anwendung in der Mitte des 20. Jahrhunderts zurück, da die Strahlenbelastung für die behandelnden Mediziner bei der manuellen Handhabung der Strahlenquellen zu hoch waren, indes ist aktuell ein größeres Interesse wieder entstanden, auch durch die Entwicklung ferngesteuerter Nachlade-Systeme (Afterloading genannt) und die Verwendung neuer Strahlenquellen, mit denen das Risiko hoher Strahlenbelastung für Arzt und Patient herabgesetzt wurden, vgl. z.B. Anspruch 14. Das Vorschieben der Strahlungsquellen in den vorgegebenen Bahnen, z.B. Katheter, führt sie zum Target, anderenfalls sind die Strahlungsquellen in einem Strahlungssafe zurückgezogen, um die Umgebung, Pfleger und Ärzte zu schützen.

Aus Teilbereichen der mehreren Paretofronten kann eine kombinierte, globale Paretofront gebildet werden, bevorzugt wird die ausgewählte Lösung entlang der kombinierten globalen Paretofront geändert.

Anspruch 16 wird durch eine Bezugnahmen hier einbezogen, ohne Bezugszeichen.

Technische Lösungen zum genannten technischen Problem finden sich auch in den Ansprüchen 20 oder 21, die den Aufbau einer grafische Benutzerschnittstelle (eine GUI, graphical user interface) beanspruchen und hier inhaltlich einbezogen sind.

Eine erste GUI arbeitet mit einer Darstellung auf einem Sichtgerät zur Ausführung des Verfahrens. Die GUI stellt auf einem Sichtgerät des Eingabe-/Ausgabegeräts für zumindest einige Technologien je eine Paretofront in einem Paretofront-Diagramm dar. Jede der Paretofronten wird Patch genannt. Jedes der Patches wird in Abhängigkeit von zumindest zwei Kriterien in einem Patchbereich des Sichtgeräts dargestellt. Auf dem Sichtgerät sind in einem Bedienbereich, außerhalb des Patchbereichs, eine Anzahl von Bedienhilfen dargestellt, von denen jede Bedienhilfe ein Kriterium repräsentiert. Die Bedienhilfen besitzen jeweils einen Selektor, wobei bei einem Bewegen oder Bedienen eines Selektors der jeweiligen Bedienhilfe in eine Richtung ein entsprechendes Kriterium verbessert wird oder in eine gegenläufige Richtung verschlechtert wird. Jede Bedienhilfe stellt mit Hilfe des zugehörigen Selektors - für eine interaktive Kommunikation mit dem Planer - eine im aktuellen Zustand ausgewählte Lösung in dem Patchbereich als Punkt zᵢ auf einer der mehreren Paretofronten dar, wobei die ausgewählte Lösung, (durchgehend) entlang mehrerer Patches veränderbar ist. Ein Unschärfeintervall ist bestimmbar (oder veränderbar festgelegt), das bei einem Zielkriterium definiert ist, welches auch im interaktiven Patchbereich durch einen optisch hervorgehobenen Streifen entsprechend repräsentiert angezeigt ist und zumindest zwei Paretofronten als zumindest zwei Behandlungstechnologien mit seiner Erstreckung erfasst. Dadurch wird interaktiv durch eine Eingabe des Planers eine Gleichwertigkeit von zwei Technologien demselben Planer vom Patchbereich so vermittelt, dass die beiden dargestellten Behandlungstechnologien einen gleichen Wert c1₁ des gleichen Zielkriteriums im jeweiligen Patch haben.

Eine andere GUI eignet sich zum Entwerfen oder Gestalten einer Therapie als Behandlungsplan, in einer eine interaktiven Navigation an einem Sichtgerät. Auf dem Sichtgerät wird für zumindest einige der Technologien je eine Paretofront zusammenhängend dargestellt, die jeweils Patch genannt wird. In einem Bedienbereich auf dem Sichtgerät sind eine Anzahl von Bedienhilfen dargestellt, von denen jede ein Kriterium repräsentiert, welches ein Bewegen oder Bedienen eines Selektors der jeweiligen Bedienhilfe erlaubt. Der jeweiliger Selektor stellt für eine interaktive Kommunikation mit dem Planer eine im aktuellen Zustand ausgewählte Lösung im Patchbereich als Punkt zᵢ auf einer der mehreren Paretofronten hervorgehoben dar, um es zu Erlauben, die ausgewählte Lösung zu verändern, entlang mehrerer Patches (durchgehend oder simultan). Der Planer verändert dazu einen jeweiligen Selektor einer jeweiligen Bedienhilfe in seiner Lage. Ein Unschärfeintervall ist vorgesehen, welches bei einem Zielkriterium definiert ist, und welches auch im interaktiven Patchbereich entsprechend repräsentiert angezeigt wird. von ihm werden zumindest zwei Paretofronten für zumindest zwei Technologien erfasst. Interaktiv kann eine Gleichwertigkeit von zwei Technologien dem Planer vermittelt werden, die der Planer ohne diese GUI nicht zu erkennen vermag.

Eine neue Verwendung eines Mauszeigers wird aus Anspruch 25 hier einbezogen, ohne Bezugszeichen.

Ausführungsbeispiele der Erfindung sind mit Hilfe der Figuren näher erläutert. Alle Erläuterungen sind gleichermaßen für die Offenbarung heranzuziehen, aber sie sind nicht so auszulegen, dass sie als notwendige Bestandteile der Ansprüche hinzugenommen werden müssen. Alle folgenden Beispiele bleiben auch dann Beispiele, wenn nicht explizit "beispielsweise" ihnen vorgelagert ist.
- Figur 1: ist eine schematische Ansicht eines Beispiels eines Ablaufs eines Verfahrens einer oder mehrerer Ausführungsformen der Erfindung. Hier mit Patches als Technologien auf einem Sichtgerät mit Display 10 (repräsentiert mit den vier gestrichelten Ecken). Hier ist ein erster Systemzustand dargestellt.
- Figur 1A: hilft bei dem Verständnis der Paretofront, im Beispiel der Paretofront 201. Erkennbar sind eine Vielzahl von Stützstellen 201₁ bis 201₁₀. Die Front ist also keineswegs durchgehend, sondern nur funktional zusammenhängend, daher ist sie in Figur 1 zusätzlich als (dünne) Linie ausgewiesen, die im Grunde keine solche ist. Die Paretofront kann ebenso eine Projektion auf dem n-dimensionalen Raum dargestellt gedacht werden, in welchem Raum sie besteht, indes grafisch nur als eine Projektion dargestellt werden kann.
- Figur 1B: veranschaulicht in zwei Teilbildern der Figur 1b1 und der Figur 1b2 erreichbare Punkte auf den mehrdimensionalen Paretofronten. Im Fadenkreuz der Figur 1b1 ist die aktuelle Stellung (der navigierte Punkt). Das schlanke, aufrecht stehende, weiße Dreieck kennzeichnet die erreichbaren Punkte. Die größere schraffierte Zone darum herum die nicht erreichbaren Punkte, eher eine projizierte Fläche. Mit hier nicht dargestellter Farbkennzeichnung kann die hier nicht sichtbare, indes vorhandene Dimension repräsentiert werden. In fünf "Punkten", als kleine Karos (Würfel) dargestellt, werden dazu Unsicherheiten repräsentiert. Die Mitte davon ist jeweils der Erwartungswert, jeweils vier Arme in jew. 90° Orientierungen zeigen jeweils als Intervall Bereiche der Unsicherheit an. In Figur 1b2 werden die Einstellungen für Figur 1b1 gezeigt, die dortigen Slider können auf zwei Arten konfiguriert sein, nach Ziel (Objective) oder Input (Eingang I1, I2 und I3). Es sollten zumindest zwei davon sein.
- Figur 1C1, 1C2: zeigen die Paretofronten mit ihren Stützstellen, die dazwischen interpoliert werden können. Erkennbar sind die Formen der Paretofronten 101 bis 501 aus Figur 1. Diese werden in der Legende Patch genannt. Der aktuell Punkt, der mit einem Kreuz dargestellt ist, wird von den Slidern in Figur 1C2 abgebildet.
- Figur 2A bis 2D: sind verschiedene Strahlengeräte 100, 200, 300 und 400 die unterschiedliche Technologien bereitstellen.
- Figur 3: veranschaulicht ein Schaubild der Bewegung der Bedienhilfen 21 und 22 in mehreren Teilbildern.
- Figur 4: veranschaulicht einen fortgeschrittenen Systemzustand auf dem Sichtgerät mit Display 10, ausgehend von dem Systemzustand der Figur 1. Zusätzlich ist hier eine optional resultierende Paretofront 801 (als resultierendes Patch 801) dargestellt (in eng gepunkteter Linienform).
- Figur 5: veranschaulicht einen noch weiteren Systemzustand, bei dem ein Ähnlichkeitsbereich 30' eröffnet wurde, beim Wert c1₁. Der Ähnlichkeitsbereich wird hier durch ein (vertikales) Streifenstück 30' umschrieben und seine Länge 30" entspricht auf der zweiten Achse als zweites Kriterium c2 dem Abschnitt zwischen den Enden 30a und 30b.
- Figur 6: ist ein noch weiterer Systemzustand, in welchem der Ähnlichkeitsbereich auf ein längeres Feld 31' erstreckt wurde, entsprechend der vergrößerten Länge 31".
- Figur 7: veranschaulicht den Systemzustand von Figur 6, wobei über ein visuelles, interaktives Feld 80 als Funktionsfeld eine der Technologien abgewählt worden ist, sodass diese Technologie im Bedienbereich 2 nicht mehr erscheint oder nicht mehr angezeigt wird.
- Figur 8: veranschaulicht den Systemzustand der Figuren 6 und 7, und es ist ein ganzer Patch, hier der Patch 501, von der Anzeige im Bedienbereich 2 ausgenommen, was zur Beschränkung von Auswahlmöglichkeiten führt, die mit den Bereichen 42" und 41" gekennzeichnet sind.

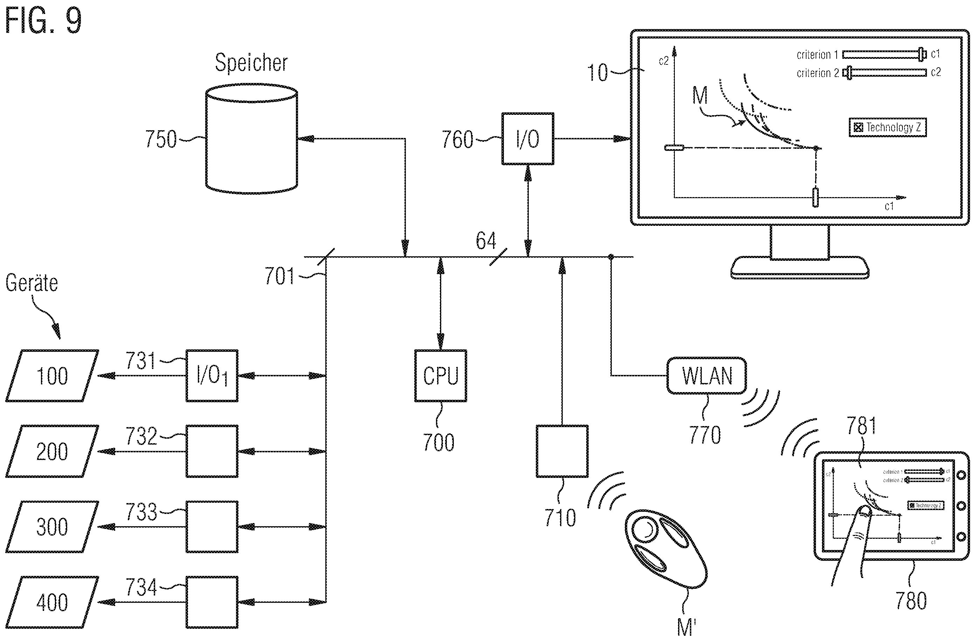

- Figur 9: veranschaulicht ein technisch-funktionelles Datensystem mit Rechner, Speichersystem und dem Eingabe-/Ausgabegerät mit einem Display 10. Das Display 10 kann ein normales Sichtgerät sein, oder ein Touch-Screen, um statt mit einem Mauszeiger mit einem Finger (als Zeigegerät) navigierende Kontrolle über den in Figur 1 abgebildeten Systemzustand zu haben. Ein "Zeiger" soll in diesem Sinne als ein "Funktionszeiger" verstanden werden, z.B. ein Mauszeiger, ein Finger oder ein Laserpointer, welche jeweils die Zeigefunktion mit der Bedienfunktion (am Ort des Zeigers auf dem Display) vereinigen.

Figur 1 veranschaulicht das Eingabe-/ Ausgabegerät, welches ein Display 10 aufweist. Dieses Display 10 ist durch die vier angegebenen Ecken symbolisch dargestellt. Es ergibt sich auch aus der Systemstruktur der Figur 9 mit der die folgend beschriebenen Funktionen realisiert werden (oder realisierbar sind).

Vergrößert dargestellt zeigt Figur 1 rechts oben einen Bedienbereich 2 und links, in größerem Flächenverbrauch als der Bedienbereich 2 einen Patchbereich 1. Der Bedienbereich 2 liegt außerhalb des Patchbereichs 1, was sagen soll, dass diese beiden sich nicht funktional behindernd überlappen. Sie erscheinen für den Planer sichtbar voneinander getrennt, wobei ihre Lage nur in dem Beispiel der Figur 1 so gewählt ist, dass der Patchbereich links unten und der Bedienbereich rechts oben (auf der Sichtfläche des Displays 10) angeordnet ist. Es besteht ein visuell sichtbares und visuell auch abgegrenztes Feld 80, welches als Funktionsfeld die Technologie Z identifiziert. Dieses Technologiefeld, welches allgemein auch Funktionsfeld 80 genannt ist, wird später erläutert und erweitert.

Der Bedienbereich 2 und der Patchbereich 1 sind funktionell miteinander gekoppelt. Diese funktionelle Kopplung soll erläutert werden. Im Patchbereich 1 werden mehrere Paretofronten gezeigt, im Beispiel sind es die Paretofronten 101, 201, ... bis 501.

Auch dargestellt im Patchbereich sind zwei Achsen c1 und c2, die senkrecht zueinanderstehen und zwei Kriterien c1 und c2 repräsentieren. Dargestellt sind das Kriterium 1 (auch c1) und das Kriterium 2 (auch c2), welche im Bedienbereich 2 sichtbar und navigierbar dargestellt sind.

Ein Schnittpunkt findet sich im derzeit ausgewählten Punkt zᵢ. Dieser ausgewählte Punkt ergibt sich durch die Einstellungen von beiden Bedienhilfen 21 und 22, welche im Bedienbereich 2 zu sehen sind. Das Kriterium 1 wird auch c1 genannt und ist horizontal im Patchbereich 1 aufgetragen. Das Kriterium 2 auch c2 genannt, ist im Patchbereich 1 vertikal aufgetragen.

Im Bedienbereich 2 werden zu den beiden Kriterien und mit den beiden Bedienhilfen 21 und 22 zwei Einstellungen angenommen, die sich durch die Bedienelemente 21a und 22a auswählen lassen und die hier auch ausgewählt dargestellt sind. Durch diese Einstellung ergibt sich bei funktioneller Verkopplung des Bedienbereichs 2 mit dem Patchbereich 1 der ausgewählte Punkt zᵢ der Technologie Z, welche Technologie Z mit 101 als Paretofront symbolisiert ist.

Zu erläutern ist dabei auch der Begriff der Paretofront. Die Paretofront ist eine Darstellung aller technischen Größen, die eine Therapie repräsentieren und per definitionem multikriteriell nicht verbessert werden können. Ein solches multikriterielles Optimum ist dadurch charakterisiert, dass es keine besseren Lösungen gibt als diejenigen, die auf der Paretofront liegen. Kein Wert (Kriterium) davon kann verbessert werden, ohne ein anderes Kriterium zu verschlechtern.

Der Begriff der Lösung ist so zu verstehen, dass eine technisch definierte Therapie als eine fraktionierte Behandlung (als fraktionierte "Sitzungen") über mehrere Tage durch einen Punkt auf der Paretofront repräsentiert wird. Diese ist demnach eine in zeitlich beabstandete Fraktionen aufgeteilte Behandlung. Im Beispiel können es zumindest 30 Tage sein, die mit einem täglichen Einsatz von weniger als eine Stunde, meist ist es deutlich weniger fraktioniert wird.

Von diesen täglichen Fraktionen können zwischen 30 bis 40 nacheinander erfolgen, meist nicht an Wochenenden und zu fest definierten Zeitpunkten an jedem Wochentag. Damit kann eine Gesamtdosis oder eine gesamte Strahlenbelastung von zwischen 60gy bis 80gy auf bis zu maximal 2gy pro Tag herabgesetzt werden.

Der Fachmann spricht von fraktionieren in Tagesdosen und kann es so ermöglichen, dem Patient eine geringstmögliche Strahlenbelastung zukommen zu lassen, bei höchstmöglicher Wirkung auf dem Target, welches durch die Bestrahlung reduziert oder, wenn möglich, ganz beseitigt werden soll.

Die Planung eines solchen Behandlungsverlaufes erfolgt durch die "Lösung", welche diesen Behandlungsverlauf für den speziellen Patient festlegt, hier repräsentiert durch einen einzigen Punkt zᵢ auf einer der Paretofronten. Repräsentativ steht dieser Punkt also nicht nur für einen Patienten, sondern für eine ganze Reihe oder Abfolge von Systemeinstellungen an den im Folgenden noch zu skizzierenden technischen Geräten.

Diese Darstellung ist eine hochabstrahierte Form der Vermittlung einer Lösung, die vorberechnet ist und in einem Speicher 750 des Bussystems nach Figur 9 vorgehalten wird.

Es ist ersichtlich, dass jeder weitere Punkt auf den Paretofronten auch eine Lösung repräsentiert, sodass die Verkettung aller Lösungen auf einer Paretofront den Verlauf dieser Paretofront definiert. Wechselt der Planer (als Benutzer des Tools zum Entwerfen und Gestalten einer Therapie als Behandlungsplan gemäß Figur 1) den Patch, so wechselt er auch das Gerät oder das Regime am gleichen Gerät, mit dem die Strahlung für den Patienten erzeugt und im Zuge seiner Therapie abgegeben wird.

Es soll hier erneut betont werden, dass nicht die Behandlung als solche patentiert wird, sondern die Planung dieser Therapie. Was indes nicht vermieden werden kann, ist die Bezugnahme auf die spätere Wirkung der Planung am und auf den Patienten, der in den folgenden Bildern mit P symbolisiert ist.

Zurück zur funktionellen Verkopplung des Patchbereichs 1 mit dem Bedienbereich 2, dies mit Blick auf Figur 1. Das Einstellen der beiden Bedienelemente 21a und 22a legt den Punkt zᵢ im Patchbereich fest. Auf den beiden Achsen der Kriterien c1 und c2 sind die Einstellungen der Bedienelemente 21a und 22a weiterhin repräsentiert, die gewählte x y-Darstellung ergibt eindeutig den Punkt zᵢ auf dem Patch 101, mit der Folge, dass dieser Plan mit seiner Technologie Z, derzeit ausgewählt ist.

Dargestellt sind also mehrere Technologien, die mit A, B, ... Z symbolisiert werden. Unter diesen Technologien können die eingangs der Anmeldung beschriebenen Technologien sein, wobei jede Technologie auch ein Regime beinhalten kann. Technologie ist also sowohl die Wahl der Strahlenart der Strahlentherapie (Radiotherapie) zu verstehen, wie auch innerhalb einer gleichen Art von Strahlen das Regime der Therapie.

Die Ziffern 1 bis 6 eingangs der Anmeldung veranschaulichen als nuklearmedizinische Strahlen die Protonen und die Photonen, jeweils mit Linearbeschleuniger angetrieben, wobei es weitere Strahlenerzeuger und in der Nuklearmedizin andere Strahlentypen oder Strahlenarten gibt. Weiterhin beschleunigte Elektronen, beschleunigte schwere Ionen, oder eben die in den Beispielen genannten Protonen und Photonen. Die Strahler können auch in der Brachytherapie eingesetzt werden. Diese Geräte verwenden zur direkten (inneren) Abgabe von Gammastrahlen vorgeschobene (radioaktivem Zerfall unterliegende) Strahlungskörperchen. Die Strahlungskörperchen können eine zylindrische Ausbildung haben, die eine Länge von weniger als 5mm haben und aus Cobalt-60, Jod-125 oder Iridium-192 gebildet sind, bevorzugt umgeben von einem Titanmantel.

Als Regime sind die Arten und Weisen gekennzeichnet, mit denen eine bestimmte Strahlenart benutzt wird, beispielsweise mit zwei Umdrehungen, beispielsweise mit neun Feldern und neun festen Winkeln, oder beispielsweise mit neun Feldern und anderen als den genannten neun festen Winkeln.

Der Bedienbereich 2 umfasst, aus der vorhergehenden Beschreibung entnommen, zwei linear bewegliche Slider als Bedienhilfen 21, 22, wobei jedem Slider derzeit ein Bedienelement 21a, bzw. 22a zugeordnet ist, der als Selektor benannt werden soll. Beispiele von Bedienhilfen sind Slider, Beispiele von Bedienelementen sind digital dargestellte Bedienknöpfe, auch Selektor genannt.

Mit diesem Selektor kann im Bedienbereich 2 ein jeweiliges Kriterium verbessert oder in der zugehörig gegenläufigen Richtung verschlechtert werden. Nach vorhandenen Konventionen ist eine Bewegung nach links eine Verbesserung, sodass abgebildet das Kriterium c2 (auf der zugehörigen c2-Achse) schon bestmöglich optimiert ist (da es am linken Rand der Bedienhilfe 22 liegt). Für das Beispiel gewendet, ist der "Selektor des Sliders nahe dem Linksanschlag".

Die entsprechende Darstellung zeigt Figur 1 im Patchbereich 1, wo Selektor 22a den niedrigsten Wert hat. Ein Erhöhen des Werts des Kriteriums c2 in der Planung führt (später, hier nicht aktive einbezogen) zu einer Verschlechterung der Therapie, aber da auch das Kriterium c1 zu beachten ist und dieses anhand der Stellung des Sliders 21 und seines Selektors 21a noch verbessert werden kann, ist mit einer Bewegung zu rechnen, die anhand der folgenden Figuren erläutert werden soll.

In einer Übersicht wird Bedienelement 21a sich nach links bewegen (auf der Achse c1 im Patchbereich 1 auch nach links). Bedienelement 22a wird sich nach rechts bewegen (auf der Achse c2 im Patchbereich 1 nach oben). Die Orientierung der Bedienhilfen 21 und 22 im Bedienbereich 2 kann auch um 90° geschwenkt sein, so dass links/rechts zu unten/oben wird. Das kann ebenso für jeden einzelnen der Bedienelemente 21 und 22 gelten, die dabei so ausgerichtet werden, dass sie wie ein kartesisches Koordinatensystem erscheinen.

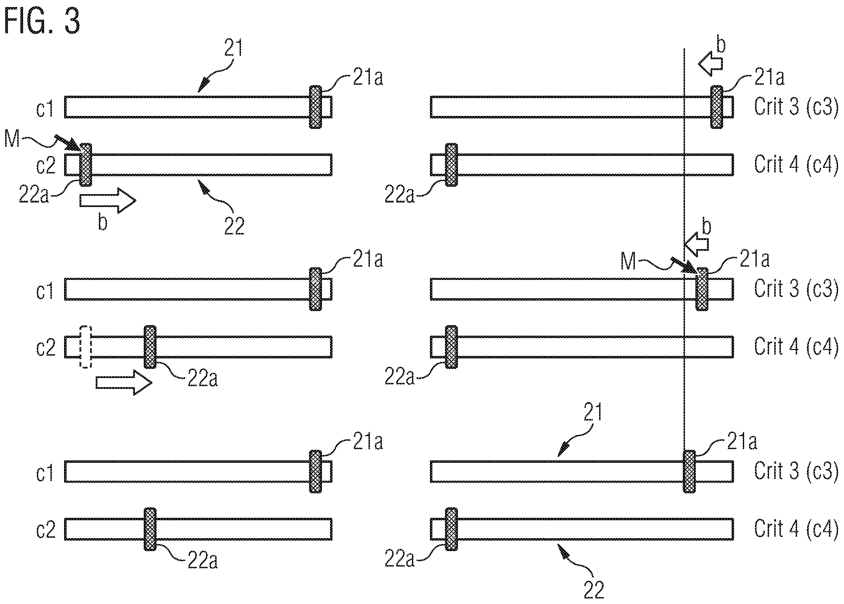

Diese Bewegung ist in Einzelschritten in der Figur 3 in sechs Teilbildern erläutert, dazu später.

Bevor diese Bewegung erläutert wird, sollen die Geräte erläutert werden, die mit den Paretofronten (Patches) 101 bis 501 der Figur 1 in Verbindung stehen, wobei vier beispielhafte Geräte gezeigt sind, die nicht abschließend sein sollen, sondern nur beispielhaft.

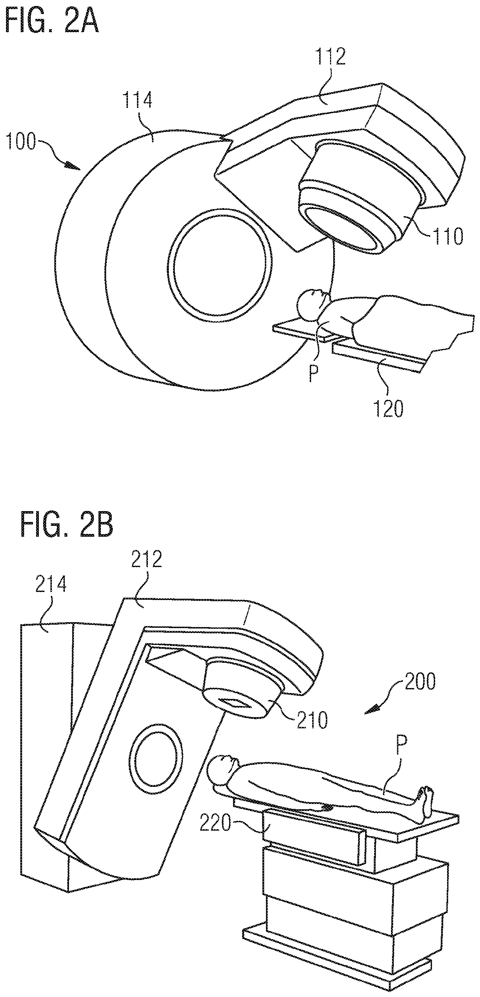

Das erste Gerät 100 der Figur 2A ist ein Gerät 100 zur Abgabe von Protonen während oder für eine Strahlentherapie eines Patienten P, der auf einem Tisch 120 (behandlungs-fertig) positioniert ist. Ein erster Tragkörper 114 ist rotatorisch gelagert und an ihm ist ein Strahlenkopf 110 angeordnet, der über eine Brücke 112 mit dem Tragkörper 114 starr verbunden ist. Über den Tragkörper kann der Winkel des Strahlenkopfes 110 eingestellt werden, der gegenüber dem Patienten P eingenommen wird. Auch die Strahlendosis und die Verteilung der Strahlung innerhalb des nicht dargestellten Protonenstrahls aus dem Strahlenkopf 110 sind einstellbar. Alle diese Werte werden in einer Paretofront repräsentiert, für einen Patienten über eine gesamte fraktionell aufgeteilte Radiotherapie. Im Beispiel kann dies der Paretofront 101 von Figur 1 entsprechen. Die Einstellung der technischen Werte des Strahlenkopfs 110 erfolgt über ein I/O-Interface 731 von dem 64bit Bus 701 aus, der in Figur 9 ersichtlich ist.

Figur 2B zeigt ein weiteres Strahlengerät, hier ein Photonenstrahlengerät 200. Der Patient P ist auf einem Sockel 220 platziert und der Strahlenkopf 210 zur Abgabe der Protonen ist an einer L-förmigen Brücke 212 angeordnet. Die Brücke 212 ist rotatorisch mit einem stationären Sockel 214 verbunden, dem gegenüber sie schwenkbar ist. Auch hier können die Einstellungen der Strahlenabgabe des Strahlenkopfes 210 vorgegeben werden, und sie für eine fraktionierte Behandlung einer Paretofront, beispielsweise der Paretofront 201 aus Figur 1, vorgegeben werden. Die Einstellungen der technischen Werte des Strahlenkopfs 210 erfolgt über ein I/O-Interface 732 von dem 64bit Bus 701 aus, der in Figur 9 ersichtlich ist.

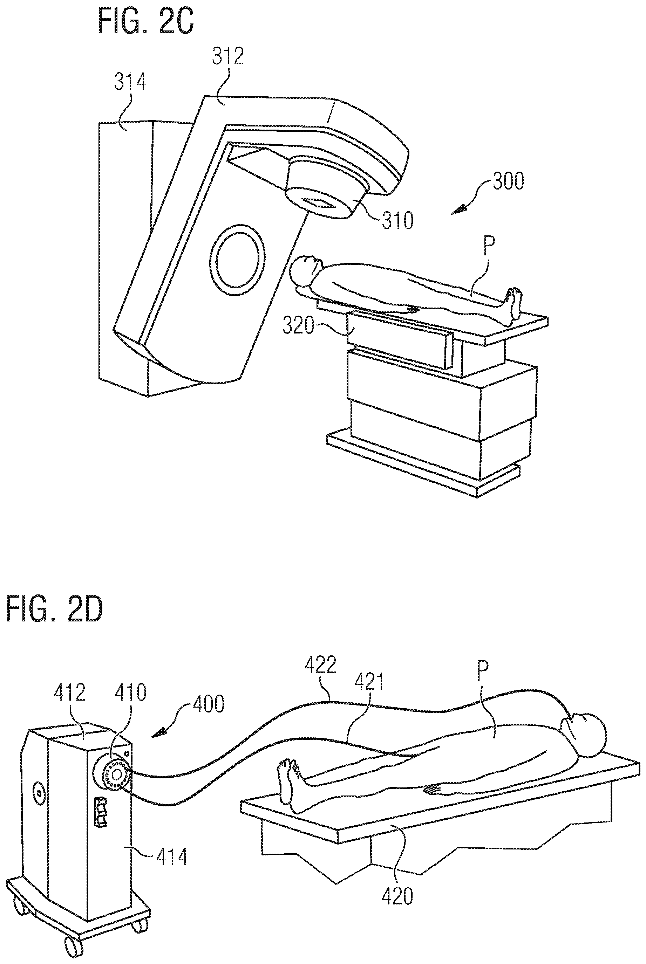

Figur 2C zeigt ein weiteres Strahlengerät 300. Auch hier ist der Patient P auf dem Tisch 320 aufgelegt. Die Brücke 312 ist schwenkbar an einem Sockel 314 angeordnet und der Strahlenkopf 310 ist ausgebildet, beschleunigte Photonen abzugeben. Die Abgabe der Photonen aus dem Strahlenkopf 310 wird so eingestellt, dass sie auf das Target und die Risiken so abgestimmt sind, dass im Rahmen der fraktionierten Behandlungssitzung primär das Target von den Strahlen in seinem Volumen erfasst wird und die Risiken von einer Strahlenbelastung abgeschirmt werden. Dazu kann eine Multilamellen-Kollimator vorgesehen sein, der in dem Strahlenkopf 310 platziert ist und dessen Lamellen so eingestellt werden, dass sie in jede beliebige Form gebracht werden, um als freien Raum zwischen sich eine passende Fläche freizugeben, die dem Zielvolumen (Target) bestmöglich entspricht. Die Lamellen selbst sind strahlen-abschirmend, beispielsweise aus Wolfram, sodass sich die Form des Strahls nahezu beliebig einstellen lässt. Zusätzlich zu dieser formgebenden Einstellung des Strahlenvolumens, eigentlich der Strahlenfläche, kann ein Regime treten, mit dem die Felder und die Winkel vorgegeben werden, unter denen die Bestrahlungen erfolgen. Die Einstellung der technischen Werte des Strahlenkopfs 310, z.B. des Multilamellen-Kollimators erfolgt über ein I/O-Interface 733 von einem 64bit Bus 701 aus, der in Figur 9 ersichtlich ist.

Ein weiteres Strahlengerät ist in Figur 2D gezeigt. Es ist ein Strahlengerät 400 zur Abgabe einer inneren Strahlung, wobei die eingangs der Anmeldung beschriebene Brachytherapie Anwendung findet und mehrere Schläuche in Form von Kathetern 422, 421 von einem Verteilerkopf 410 (auch "Strahlenkopf" genannt) ausgehen und an einen jeweiligen Strahlenort gelegt sind, der im Patienten P liegt. Der Patient P liegt auf einem Behandlungstisch 420. Aus einem Strahlensafe 412, der im eigentlichen Gerätevolumen 414 strahlensicher abgegrenzt wird, werden kleine Strahlenkörperchen, hier nicht dargestellt, herausgefahren und entlang der Katheter 422, 421 an den Ort gebracht, wo die Strahlen als innere Strahlenquelle im Patienten für eine bestimmte Zeit lang aufgebracht werden sollen. Danach werden sie zurückgeholt und wieder in den Strahlensafe 412 eingeschlossen, um eine parasitäre Abgabe von Strahlen zu blockieren. Die Einstellung der technischen Werte des Strahlenkopfs (z.B. die Zeiten des Verweilens im Body) erfolgt über ein I/O-Interface 734 von dem 64bit Bus 701 aus, der in Figur 9 ersichtlich ist.

Das Strahlenabgabe des Strahlengeräts der Figur 2D kann in ihrer Planung beispielsweise der Paretofront 501 von Figur 1 entsprechen. Das zuvor beschriebene Strahlengerät 300 der Figur 2C kann seine Strahlenabgabe in seiner Planung beispielsweise der Paretofront 301 entsprechen.

Weitere Geräte, hier nicht gesondert dargestellt, können vorgesehen sein und in ihrer Strahlenabgabe Gegenstand von Paretofronten werden, so präsentiert durch die Paretofront 401 von Figur 1.

Die Figur 3 veranschaulicht in (angenommener) Zeitlupe oder in kleinen Schritten die Veränderung der Bedienelemente 21a und 22a der beiden Kriterien c1 und c2. Dazu sind drei Teilbilder in der linken Hälfte der Figur 3 repräsentativ. Ein Funktionszeiger M wird angenommen, der später genauer erklärt ist.

Ausgehend von einer Einstellung der Bedienelemente 21a und 22a an den Bedienhilfen 21 und 22 ist der Zustand anfangs gezeigt, der in Figur 1 abgebildet ist. Das Kriterium c2 ist bestmöglich eingestellt, da am weitesten links, Im Beispiel soll eine Verschlechterung dieses Wertes durch die Bewegung b herbeigeführt werden, wobei durch einen Mauszeiger M oder bei einem berührempfindlichen Bildschirm 780 nach Figur 9 per Aktivieren und Wischen mit dem Finger das Bedienelement 22a nach rechts verschoben werden soll. Es verschiebt sich dabei in der mittleren Darstellung von seinem ursprünglichen Ort im oberen Teilbild zu einem Ort, der weiter rechts liegt. Dies kann mit einer optischen Fading Technik dargestellt werden, der bisherige Ort verbleicht, der neue Ort entsteht.

Die Bewegung b ist abgeschlossen oder beendet, wenn das Bedienelement 22a seine neue Position erreicht hat, wie in dem dritten Teilbild auf der linken Seite zu sehen ist. Die ursprüngliche Lage, die im mittleren Teilbild noch angedeutet war, ist dann nicht mehr zu sehen. In einer Bewegung vom ersten zum dritten Teilbild gibt es viele Zwischenzustände, die hier nicht dargestellt sind.

Im Beispiel ist angenommen, dass das Kriterium c1 sich bei einer solchen Veränderung der Bedienhilfe 22a nicht verändert, was praktisch nicht der Fall sein wird, da sich bei einer Veränderung des einen Kriteriums auch das andere Kriterium mit verändern wird, da diese funktionell über die Paretofront 101 miteinander verbunden sind, also funktionell miteinander gekoppelt sind, obwohl sie nicht physisch miteinander verbunden sind.

Die eigentliche funktionelle Verbindung findet durch die Vorgabe der vordefinierten Lösungen auf der Paretofront, dem Patch 101 statt, verändert der Planer eine Lösung in einem Kriterium, verändert sich auch ein zweites, drittes und/oder viertes Kriterium und damit auch das zweite (oder auch weitere) Bedienelement(e) auf eben diesem zweiten Kriterium oder für eben dieses zweite Kriterium c1.

In Figur 3 sind in der rechten Hälfte zwei andere Kriterien c3 und c4 abgebildet. Hier ist eine Bewegung des Kriteriums c3 von rechts nach links zu sehen, also hin zu einem besseren Wert. Auch hier ist zur Veranschaulichung das Kriterium c4 mit dem Bedienelement 22a unverändert dargestellt, dies repräsentiert durch die gleich-bleibende Position des Bedienelements 22a im Slider 22.

Die Bewegung b verläuft dargestellt langsam und schrittweise bis hin zu der eingezeichneten vertikalen Hilfslinie und der neuen Position des Bedienelements 21a auf der Bedienhilfe 21 (auch als Selektor und Slider, die Beispiele davon sind). Der Funktionszeiger M nimmt hierbei das Bedienelement 21a der Bedienhilfe 21 vom Planer gesteuert mit und stellt es auf die Position des dritten Teilbildes ein.

Unter Berücksichtigung der Optionen, dass mehrere Kriterien über Bedienhilfen verändert werden können, in den hier gewählten Beispielen aber nur zwei Kriterien c1 und c2 vertieft erläutert werden, kann unter Zuhilfenahme der Figur 3 auch verstanden werden, dass zwei weitere Kriterien c3 und c4 verändert werden können, oder aber die Kriterien c1 und c4 im Bedienbereich 2 dargestellt sind, funktionell verkoppelt mit den dann im Patchbereich 1 passenden Darstellungen der Paretofronten 101 bis 501 (in den Achsen c1 und c4).

Typischerweise werden fünf bis sechs Kriterien verglichen. Die Paretorechnungen zur Vorgabe der vordefinierten Lösungen benutzen zwischen zehn und fünfzehn Kriterien. Im gegenseitigen Wettbewerb stehen drei bis fünf Kriterien, zwischen denen ein Kompromiss zu finden ist, um eine Lösung zu finden und festzulegen, mit der der Planer so zufrieden ist, dass er diesen Plan dem Arzt zur Überprüfung und abschließenden Bewilligung vorlegt.

Die Kriterien liegen funktionell darin, die Risiken in unmittelbarer Umgebung zu schützen und den Tumor für den Fall einer Krebstherapie bestmöglich zu schädigen, also bestmöglich mit Strahlung zu zerstören. Die Bereiche in weiterer Entfernung von dem Tumor sind weniger kritisch. Oft bestehen drei Entscheidungskriterien daraus, was das Rektum, die Prostata und die Blase als Strahlendosis erhalten, und ob ein vorgegebener Plan für einen Patienten gut ist.

Beschrieben war, dass eines der Kriterien die Behandlungszeit sein kann. Die Behandlungszeit spielt eine Rolle für den für eine Therapie angemeldeten Patienten, und es spielte eine Rolle, wie gut diese Therapie für den Patient ist, also wie sehr die Zeit reduziert werden kann, die ein Patient beispielsweise zu warten hat, bevor er die für ihn bestmögliche Therapie erhalten kann. Hier soll die Güte der Therapie auch einbeziehen, dass der Patient eine geringstmögliche Wartezeit erfährt, wenn er auf die für ihn erarbeitete Therapie wartet (time to treatment). Das geht Hand in Hand damit, dass die vorhandenen Geräte (Strahlengeräte) so benutzt und eingesetzt werden, dass für den Patient das vorhandene Gerätepotential ausgeschöpft werden kann, und nicht bestimmte Geräte bevorzugt werden, auf denen oder mit denen mehrere Patienten zu behandeln sind. Dies wird zu Engpässen führen, wenn nicht andere Geräte gleichermaßen oder in zumindest sehr ähnlicher Weise die fraktionierten Zeitelemente einer Therapie ausführen können, oder könnten.

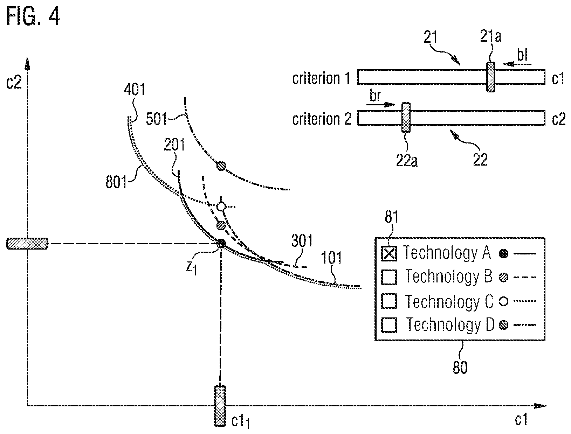

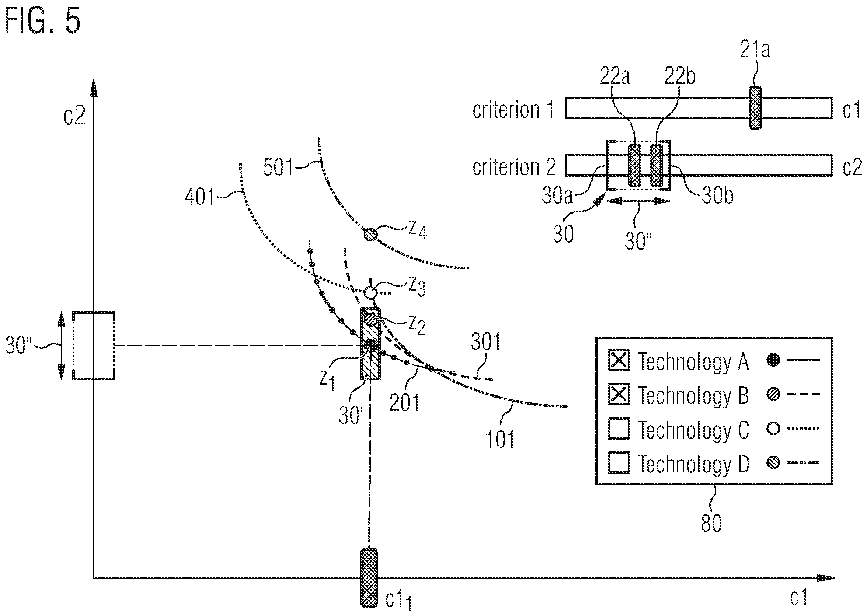

Um dies zu erreichen, findet sich in Figur 5 ein Unschärfeintervall 30 der Länge 30" (als hier dargestelltes vertikales Streifenstück 30' im Patchbereich 1), welches für ein Kriterium, hier c2 eingeführt wird. Um diese Unschärfe besser zu verstehen, soll zuerst die Figur 4 erläutert werden, bei der noch kein solches Unschärfeintervall 30 eingeführt ist, indes eine resultierende Paretofront 801 als zusätzliche Option.

Die resultierende Paretofront 801 verwendet Abschnitte anderer dargestellter Paretofronten (Patches 101, 201 und 401) für drei dargestellte Technologien. Oder sie verläuft entlang dieser Abschnitte. Sie ist eine grafische Option - auch die Summe der Patches selber, hier 101, 201 und 401 erfüllt diese Aufgabe, da es funktionell zugelassen sein soll, über die Grenzen einer Technologien hinaus zu navigieren.

In dem Beispiel der Figur 4 enthält die dargestellte resultierende Paretofront 801 Abschnitte der Paretofronten 401, 201 und 101. Die resultierende Paretofront 801 ist die Menge der Pareto-Optima (Pareto-Menge) der Technologien. Die resultierende Paretofront 801 repräsentiert also die Pareto-Optima der betrachteten Pareto-Optima der Technologien.

Die resultierende Paretofront 801 kann ebenso zu den folgenden Figuren 5 bis 8 hinzu gedacht werden, wo sie nicht zusätzlich eingezeichnet wurde. Für eine verbesserte Übersichtlichkeit ist sie in Figur 4 graphisch leicht von den Paretofronten der Technologien abgesetzt. Dies kann sich auch in einer Darstellung auf einem Display/Sichtgerät 10 empfehlen.

Der Planer hat in Figur 4 durch Verlagern des Bedienelements 21a nach links für das Kriterium c1 einen verbesserten Wert eingestellt. Es ergibt sich, aufgrund der funktionellen Verkopplung und dem Verlauf der Paretofronten, eine Verlagerung nach rechts für das Kriterium c2, wobei automatisiert über die funktionelle Verkopplung das Bedienelement 22a nach rechts verlagert wird, während das Bedienelement 21a vom Planer durch Mausklick oder per Berührung und Wischen/Schieben nach links verschoben wird. Die beiden Pfeile br und bl zeigen diese Bewegung. Die Bewegung war veranschaulicht anhand der Figur 3, dort bei aufgehobener funktioneller Verkopplung und Bewegung von nur einem Bedienelement an einem Kriterium.

Es zeigt sich in der Figur 4, dass durch Bewegen (Verändern des Kriteriums c1 auf einen verbesserten Wert), der in dem Patchbereich 1 angesteuerte Punkt z₁ ein anderer ist. Durch die Bewegung des Bedienelements 21a nach links wandert der Punkt z₁ auf der Paretofront 201 von rechts (weiter unten) nach links (weiter oben). Im Beispiel repräsentiert an der Stelle, wo die beiden Bedienelemente 21a und 22a im Bedienbereich 2 auf den Achsen der Kriterien c1 und c2 zu liegen kommen.

Was unter Zuhilfenahme der Figur 1 an der Figur 4 besser nachvollzogen werden kann, ist die Bewegung des allgemein positionierten Punktes zᵢ hin zum hier dargestellten Punkt z₁. Der Patch wurde gewechselt von der Paretofront 101 (dem Patch 101) auf die Paretofront 201 (dem Patch 201).

In Figur 4 ist dann auch symbolisiert, dass an der Stelle c1₁, die vom Bedienelement 21a im Bedienbereich festgehalten wird, mehrere Punkte existieren, die auf anderen Patches liegen, so die Punkte auf den Patches 301 und 101 (bzw. 401). Nachdem diese Patches aber nicht ausgewählt sind (sh. das Funktionsfeld 80), bleibt es bei der Technologie A, die dem Patch 201 zugeordnet ist (und die beiden Kriterien c1 und c2 definiert).

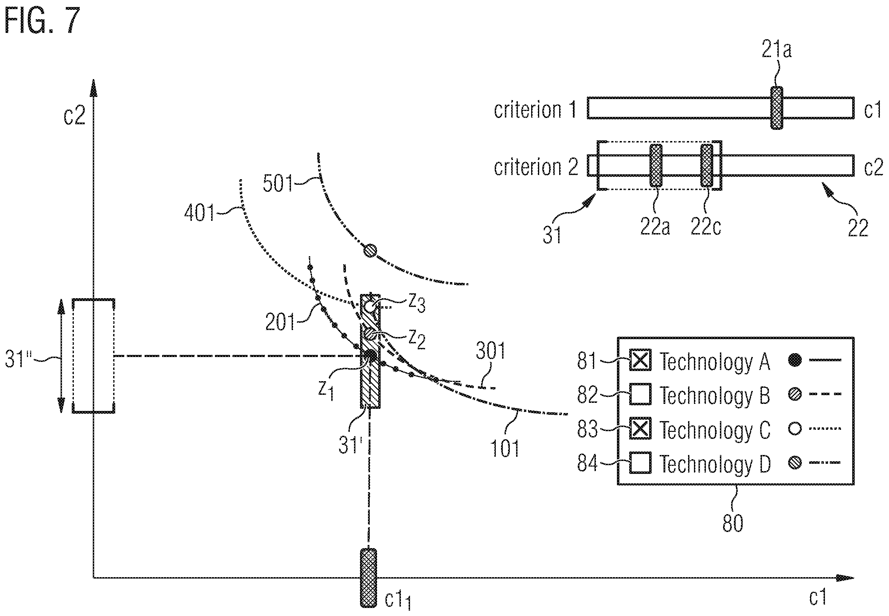

Das definiert das Funktionsfeld 80, welches eine Anzahl von Technologien repräsentiert, die vom Planer ausgewählt und abgewählt werden können. Diese Auswahl ist durch ein x in einem Kontrollkästchen 81 symbolisiert (sh. Figur 7). Auch symbolisiert ist die Zuordnung der z-Punkte durch die passende Einfärbung (Schraffur) der Punkte und der zugehörigen Bahnen, auf denen sie sich bewegen können, wobei jede Bahn eine Paretofront ist. Jede Bahn hat eine eigene Linienform, auch im Funktionsfeld 80 dargestellt, und dazu gehört eine eigene Schraffur des Punktes auf der Bahn. Punkt z₁ ist ausgefüllt und auf der durchgezogenen Bahn, die dem Patch 201, und der Technologie A entspricht. Sie ist ausgewählt und auf ihr kann vom Planer navigiert werden.

In dem Funktionsfeld 80 als visuellem Feld kann die Vorgabe der anzuzeigenden Technologien erfolgen.

Die Auswahl von weiteren Technologien führt anhand der Figur 4 aber nicht dazu, dass mehrere Patches angewählt werden können und damit mehrere Technologien bei einer Einstellung zur Verfügung stehen, oder aber zur Navigation auswählbar sind. Dazu bedarf es der zunächst angedeuteten Funktion der Erweiterung von einer Koordinate auf einer einzelnen Zielgröße (einem Zielkriterium) auf ein Intervall auf zumindest einer der Achsen, hier Kriterium c1 oder c2.

Diese Einstellung ist in der Figur 5 erfolgt. Die Einstellung zeigt den gleichen Zustand wie denjenigen von Figur 4 mit den drei möglichen Zielpunkten z₁, z₂ oder z₃. Von diesen drei möglichen Technologien sind in dem Funktionsfeld 80 zwei ausgewählt, sodass zwei Technologien im Ähnlichkeitsbereich des Unschärfeintervalls 30 liegen, welches die einzelne Koordinate des Kriteriums c2 auf einen Koordinatenbereich erweitert. Der erweiterte Bereich der Länge 30" ist das Unschärfeintervall (auch Unschärfebereich) mit einer unteren Grenze 30a und einer oberen Grenze 30b. Nachdem die Konvention eine Verbesserung nach links verlangt, sind die Begriffe des oberen und unteren Bereichs missverständlich, sodass sie linkes Ende 30a und rechtes Ende 30b genannt werden sollen. Zwischen diesen beiden erstreckt sich der Unschärfebereich 30 mit einer Länge 30".

Das Unschärfeintervall beim Kriterium c2 ist grafisch auch im Patchbereich 1 sichtbar dargestellt, auf der Kriteriumsachse c2.

Die Länge 30" entspricht dem Intervall zwischen linkem Ende 30a und rechtem Ende 30b in dem Bedienbereich 2. Auch abgebildet ist dieser Bereich über das sich vertikal erstreckende (schraffiert dargestellte) Feld 30', welches im gezeigten Beispiel zwei Technologien, die Technologie A und die Technologie B erfasst (oder überspannt). Durch Auswahl dieser beiden Technologien im Funktionsfeld 80 ergibt sich für den Planer eine weitere Option. Er kann statt der Technologie A entlang der Paretofront 201 auch diejenige Technologie B auswählen, die entlang der Paretofront 301 verläuft, da sie der erstgenannten Technologie A und der Paretofront 201 sehr ähnlich ist.

Die linke und rechte Grenze der erweiterten Identität, also jetzt der Ähnlichkeit kann über eine separate Einstellung, die nicht bildlich wiedergegeben ist, erfolgen. Dies kann eine weitere Bedienhilfe sein, die eine Schwelle definiert, die unterhalb von 10% liegen kann, bevorzugt auch liegen sollte. Alternativen sind die Festlegung durch Mausklick oder die Festlegung über Double-Tap und Wischen auf einem Tablet, also bei einem touch-sensitiven Ausgabe/Eingabegerät 780 als Eingabe-/Ausgabegerät 10.

Device-übergreifend soll von einem "Funktionszeiger" gesprochen werden, der Pointer (als Zeiger) und Funktionsoption vereinigt. Die Maus zeigt mit einem Symbol, das Festhalten eines Clicks am Display erlaubt eine Funktion "Verschieben", oder mit einem Doppelklick eine Anwahl. Ebenso arbeitet der Single tap oder der double tap eines Fingers als Locator oder Activator auf oder an einem Screen-enabled device (z.B. einem Tablet). Ebenso auch der Laserpointer als ein Zeigegerät, das mit eine Funktion gekoppelt ist, durch on/off oder das Zeichnen eines Symbols am Ort des Zeigers, z.B. als kleiner Kreis mit einer 360° Bewegung im Uhrzeiger- oder Gegenuhrzeigersinn.

Auch dargestellt in Figur 5 ist das Erscheinen des zweiten Patches 301 und des von der Unschärfe erfassten weiteren Punktes z₂ durch Darstellen eines zweiten Bedienelements 22b. Beide Bedienelemente 22a und 22b liegen innerhalb der beiden Grenzen 30a, 30b der erweiterten Identität oder des Ähnlichkeitsbereichs 30".

Erwähnt war bereits, dass das auf die Länge 30" erweiterte Feld 30' als Unschärfeintervall 30 im Patchbereich 1 zu dem schmalen Feld 30' führt, hier schraffiert dargestellt, welches zumindest zwei Paretofronten erfasst. Nachdem der Planer auch die Spannbreite oder die Größe der Unschärfe in der Hand hat, kann er durch Verengung und Erweiterung dieses Unschärfeintervalls 30 erreichen und auch interaktiv sehen, ob er in der Planung einen Spielraum hat. Ist die Unschärfe zu gering, erscheint in seinem Bedienfeld kein weiteres Bedienelement. Fasst er die Unschärfe größer, wie im dargestellten Beispiel mit den beiden Enden 30a und 30b, erhält er zumindest einen weiteren Patch zur Auswahl und damit eine weitere Technologie, wie auch im Funktionsfeld 80 zu sehen.

Nicht erreicht werden kann die Technologie entsprechend der Paretofront 401 oder der Paretofront 101, da der zugehörige Zielpunkt z₃ außerhalb der erweiterten Identität liegt, also außerhalb des Unschärfeintervalls 30'.

Das Unschärfeintervall kann durch einen weiteren, in Figur 9 nicht dargestellten Slider oder einen auf dem Display funktionell abgebildeten und optisch bedienbaren Dreheinsteller vorgegeben werden. Auch kann über ein Feld von Kontrollkästchen ein diskret vorgegebener Wertebereich von zum Beispiel 5%, 6%, 7%, 8% und 10% sowie 15% vom Planer auswählbar sein. Das Anklicken eines anderen Prozent-Bereichs, als derzeit eingestellt, löscht die Einstellung des bisherigen Unschärfeintervalls.

Nach der beschriebenen Spezifikation der klinischen Ziele und der Festlegung einer Liste von möglichen Technologien, wurde automatisch ausgerechnet, wie die Paretofronten liegen, die in den Figuren dargestellt sind. Dem Planer werden alle Technologien zur simultanen Navigation angeboten, und er hat im Wege der interaktiven Bedienfähigkeit die Möglichkeit, den Identitätsbereich zu verlassen, hin zu einem Ähnlichkeitsbereich, der Unschärfe genannt wird, um mehrere Technologien gleichzeitig zur Auswahl zu haben. Dazuhin ist ersichtlich, dass der Planer von einem Patch zum nächsten wechseln kann, er also von einer Paretofront zu einer anderen Paretofront wechselt und damit nicht auf eine Technologie und eine Paretofront beschränkt ist. Dadurch ergibt sich die Möglichkeit, eine nicht-dominante Menge der Vereinigung aller Patches zur Navigation zur Verfügung zu haben.

Im praktischen Sinne würde die Figur 5 für einen Planer die Bedeutung haben, dass ein Patient P nicht nur mit der Technologie entsprechend der Paretofront 201 und der Therapie z₁ behandlungsfähig ist, sondern im Unschärfeintervall 30 auch mit der Therapie z₂ auf einem anderen Gerät oder demselben Gerät mit einem anderen Behandlungsregime bedient (behandelt) werden kann.

Insgesamt wird damit für eine Klinik nicht nur der einzelne Patient relevant, sondern die Summe aller Patienten, die mit fraktionierten Therapien über einen längeren Zeitraum von beispielsweise 30 bis 40 Tagen behandelbar sind. Mit Blick auf die Klinik ist dies auch nicht auf die 30 bis 40 Tage beschränkt, sondern kann über das ganze Jahr geplant werden, und jeder der Vielzahl von behandelten Patienten wird über das Unschärfeintervall so in den gesamten Gerätepark (der verfügbaren Strahlengeräte) des Krankenhauses eingeplant, dass nicht nur das Beste und optimalste Gerät, was meist das aktuell erworbene, technisch fortschrittlichste Gerät ist, verfügbar ist und benutzt wird, sondern auch viele andere Geräte über das beschriebene Unschärfeintervall für die Patienten oder den speziellen Patienten zur Verfügung stehen, was die Gesamtgüte seiner Therapie verbessert, da die Wartezeit geringer wird, bis zum Beginn seiner Behandlung.

Das Funktionsfeld 80 kann auch die Funktion eines Previews haben, dies soll mit der Figur 5 ersichtlich werden, in der vier Technologien A bis D im Funktionsfeld 80 angeboten werden.

Diese mehreren, bspw. vier Technologien sind diejenigen, welche über den Wert c1₁ möglich sind, weil sie alle von diesem Wert erreicht werden, also die Zielpunkte z₁ bis z₄. Alle diese Technologien erscheinen automatisch gesteuert von der zugehörigen Software in dem erweiterten Funktionsfeld 80, verglichen zur Technologie Z aus Figur 1. Der dortige Punkt zᵢ und die Position des Bedienelements 21a lassen keine weiteren Schnittpunkte zu, sodass auch keine weiteren Paretofronten auswählbar sind, anders als in Figur 5, in der sich durch die Position des Bedienelements 21a (mit Kriterium c1) vier Möglichkeiten anbieten, bei indes unterschiedlich großem Unschärfeintervall 30.

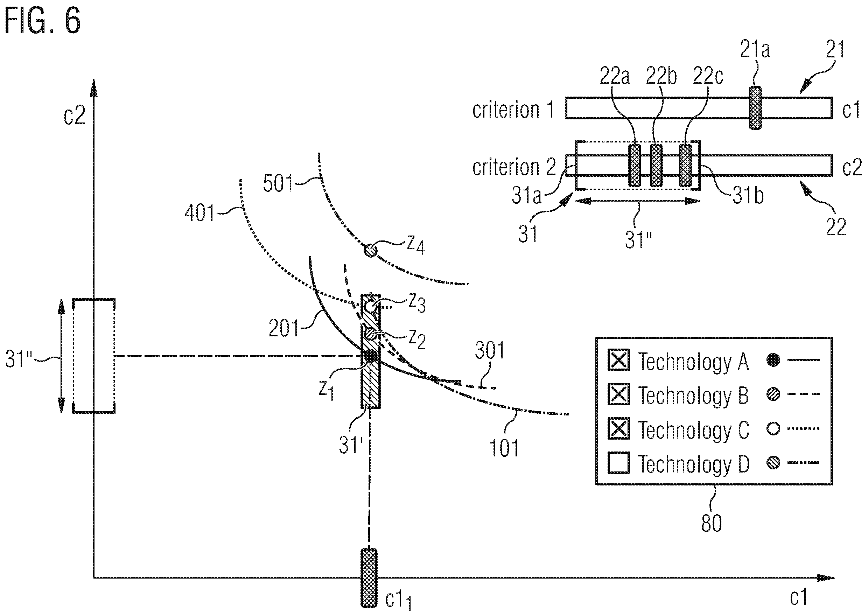

Figur 6 veranschaulicht den Systemzustand der Figur 5, und es ist ein erweitertes Feld 31' vorgesehen, welches durch Verschieben der beiden Grenzen 31a und 31b im Slider 22 entsteht. Die Spannweite dieses erweiterten Ähnlichkeitsbereichs 31", wie auch auf der Achse c2 des zweiten Kriteriums c2 abgebildet, ist größer als die Spannweite 30" in Figur 5.

Es werden mehr Paretofronten erreicht und es ergeben sich mehr Schnittpunkte z₁, z₂ und z₃. Diese Punkte, Zielpunkte oder Schnittpunkte werden interaktiv und visuell auch auf dem Slider oder in dem Slider für das Kriterium c2 angezeigt. Dort entstehen zwei weitere Bedienelemente 22b und 22c bei schon vorhandenem Bedienelement 22a, welches den Schnittpunkt z₁ repräsentiert.

Der erweiterte Ähnlichkeitsbereich erweitert den ersten Ähnlichkeitsbereich der Figur 5. Figur 5 selber war bereits ein erster Ähnlichkeitsbereich, der weitergehend als die Identität der Abbildung der Figur 4 reichte, um mehrere Technologien zu erfassen. All diese Erweiterungen, die über eine einzelne Technologie hinausgehen, werden von der Unschärfe als Intervall begrifflich erfasst, das in sich auch weiterhin steuerbar ist, wie im Folgenden beschrieben wird.

Das Unschärfeintervall 31 mit der Erstreckung 31" aufgrund des vertikalen Feldes 31' an dem Zielwert c1₁ der Kriteriumsachse c1 kann, wie bereits zuvor erläutert, auch verändert werden, es kann größer oder kleiner gestellt werden, was der Planer vornehmen kann. Diese Veränderung kann durch Anklicken und Ziehen der Grenzen 31a oder 31b, individuell oder gemeinsam erfolgen. Es kann auf einem Touchscreen 780 dadurch erfolgen, dass eine Grenze durch einen ersten Touch aktiviert wird (Tap) und danach durch ein Wischen verschoben wird (Wipe oder Swipe).

Die Grenzen, eine oder beide, können auch durch einen separaten Slider, linear oder gekrümmt ausgebildet, verändert werden. Dieser Slider ist nicht gesondert dargestellt, sondern in das Fachverständnis des Lesers gestellt.

Allen Varianten ist gemeinsam, dass die Länge 31" verändert wird.

In Figur 6 war sie bereits gegenüber der Figur 5 vergrößert worden. Durch das Vergrößern erscheinen in dem Funktionsfeld 80 mehr Technologien, das als "funktionell verkoppeltes" Funktionsfeld Bestandteil der Gesamtdarstellung auf dem Display/Bildschirm 10 ist. So kann es Einfluss nehmen, und zwar auf die Interaktionsfähigkeit mit dem Planer.

Das Funktionsfeld 80 kann nach einem Erweitern des Ähnlichkeitsbereichs 31 weitere Technologien anzeigen. Im Beispiel wird hier unter den Technologien A, B und C auch die erreichbare Technologie D in das Funktionsfeld aufgenommen.

Dieser Schnittpunkt ist in dem Patchbereich 1 mit z₄ benannt und liegt auf der Paretofront 501. Aktuell kann er von der zweiten Bedienhilfe 22 nicht erreicht werden, hier sind nur drei Bedienelemente 22a, 22b und 22c als jeweiliger Selektor vorgesehen. Dennoch zeigt das Funktionsfeld 80 schon vorab an, dass eine weitere Technologie zur Verfügung steht, die aufgrund der beschränkten Länge des vertikalen länglichen Felds 31' nicht erreicht wird.

In dieser Hinsicht ist das Funktionsfeld 80 also ein Indiz, was noch sein könnte, was dem Planer interaktiv zur Kenntnis gebracht wird, was aber aktuell durch seine Interaktionen in dem oder mit dem Bedienfeld und hier besonders durch die (begrenzte) Erstreckung des Unschärfeintervalls 31 nicht zum Tragen kommen kann.

Hypothetisch kann angenommen werden, dass eine weitere Verschiebung der Grenze 31b dazu führen würde, dass auch der Patch 501 erreicht wird. Das könnte die Ähnlichkeit indes überstrapazieren. Bevorzugt liegt der gewählte Bereich der Ähnlichkeit unterhalb von 10%-Punkten. Er ist also sehr eingeschränkt, um die Ähnlichkeit von der funktionellen Bedeutung her zu erhalten. Es mag in Erinnerung gerufen werden, dass eine Ähnlichkeit, die hier interaktiv zum Auffinden von Alternativen von Behandlungsplänen verwendet wird, technisch bedingt ist, also eine technisch sinnvolle Ähnlichkeit gesucht wird, die Auswege aus bestehenden Constraints eröffnet. Diese Auswege würde ein normaler Planer nur aus den bekannten Geräten, die am Therapieort (meist dem Krankenhaus) zur Verfügung stehen, nicht erahnen können. Es ist ihm auch fremd, welche technischen Ähnlichkeiten ein Protonengerät, ein Neutronengerät und/oder ein Photonengerät hat oder haben könnten, ohne die Darstellung beispielsweise der Figur 6 zu verwenden, welche die zugehörigen Paretofronten 101 bis 501 zur interaktiven Navigation zur Verfügung stellt.

Zuvor war erläutert, wie anhand der Figur 6 der Ähnlichkeitsbereich 31 als Unschärfe(intervall) vergrößert wird. In diesem vergrößerten Ähnlichkeitsbereich stehen dem Planer aber weitere Optionen offen, hier zum Beispiel diejenige Option, dass eine bestimmte Technologie, die eigentlich im Rahmen der Ähnlichkeit als verfügbar dargestellt wird, nicht ausgewählt werden soll.

Hierzu soll die folgende Figur 7 erläutert werden.

Figur 7 ist der Systemzustand aus Figur 6. Die Differenzen zur Darstellung der Figur 6 werden hier erläutert. Es bleibt bei dem gleichen Ähnlichkeitsbereich 31, der eine in Figur 7 ebenfalls ersichtliche vertikale Erstreckung 31" aufweist. In diesem Ähnlichkeitsbereich mit der genannten Erstreckung befinden sich im Bedienbereich 2 auf dem Bedienelement c2 zwei Bedienelemente 22a und 22c. Es sind also hier keine drei Elemente 22a, 22b und 22c aus Figur 6 dargestellt, sondern das Bedienelement 22b ist weggefallen, weil der Planer in dem Funktionsfeld 80 die Technologie B im Interaktionsfeld 82 deaktiviert oder "weggeklickt" hat.

Das Kontrollkästchen 82 für die Technologie B, repräsentativ für die Paretofront 301 und den Schnittpunkt z₂ fällt aus den interaktiven Möglichkeiten des Planers heraus. Dem Planer stehen die Technologien der noch verfügbaren Schnittpunkte z₁ und z₃, aus der Position der Bedienelemente 22a und 22c zur Auswahl. Für die Technologie des Schnittpunkts z₃ könnte er sich bevorzugt der Paretofront 401 bedienen und das zugehörige Gerät auswählen, da diese Paretofront im Schnittpunkt z₃ eine geringere Steigung besitzt, was ein Kriterium zur Auswahl der zugehörigen Paretofront sein kann.

Ersichtlich wird aus dieser Erläuterung auch, dass alle Elemente der Bilddarstellung miteinander funktionell verkoppelt sind.

Ein Auswählen einer Technologie oder ein Abwählen einer Technologie im Funktionsfeld 80 wirkt sich auf die Anzahl und das Vorhandensein der Bedienelemente im Bedienbereich 2 aus. Das Verändern des Ähnlichkeitsbereichs 31 wirkt sich auf die in dem Patchbereich 1 zu sehende vertikale Erstreckung des Unschärfeintervalls 31' aus, und damit auf die Anzahl der möglichen, und dargestellten Bedienelemente 22a, 22b und 22c, die wiederrum als ausgewählt oder abgewählt dargestellt oder nicht dargestellt werden, je nach Kennzeichnung im Kontrollkästchen des Funktionsfelds 80.

Die Kontrollkästchen sollen als Interaktionsfelder mit 81 bis 84 benannt werden, ausgewählt sind in Figur 7 die Kontrollkästchen 81 und 83, entsprechend den Technologien A und C und demzufolge den Bedienelementen 22a und 22c der Bedienhilfe 22 für das Kriterium c2.

Auch wenn in den Figuren nur zwei Kriterien c1 und c2 repräsentiert werden, sind mehr und andere Kriterien darstellbar, wie es anhand der Figur 3 erläutert war.

Die funktionelle Verknüpfung des Bedienbereichs 2 und des Patchbereichs 1 wie auch des Funktionsfelds 80 war zuvor erläutert und soll hier erhärtet werden. Es ist dabei nicht nur eine Bedienung über die Bedienelemente 22a oder 22c möglich, mit der über die funktionelle Verknüpfung auf einem jeweiligen Patch (sprich einer jeweiligen Paretofront) eine Bewegung erfolgen kann, hier des ausgewählten Punktes von beispielsweise z₂ bei Bewegen des Bedienelementes 22c. Dieser Punkt bewegt sich nicht horizontal, sondern in Konformität mit allen anderen Kriterien, hier in Konformität mit dem Kriterium c1, also auf der Paretofront 301. Die Paretofront ist auch in allen anderen Kriterien sichtbar, wird nur hier aufgrund der Zweidimensionalität nicht zusätzlich dargestellt, ist aber (für den Fachmann) gut vorstellbar.

Die Bedienung oder Veränderung der Lage des Bedienelements 22c kann auch dadurch erfolgen, dass der z-Punkt selbst mit einem Mauszeiger oder bei einem Touch-Panel durch aktiven Eingriff einer Wisch- oder Verschiebebewegung in seiner Lage verändert wird. Die funktionelle Verkopplung sorgt dabei dafür, dass dann auch das zugehörige Bedienelement 22c in seiner Lage auf der Bedienhilfe für das Kriterium c2 verändert wird.

Die soeben beschriebene Veränderung betrifft den Zielpunkt z₃, da er über die Technologie C und das Kontrollkästchen 83 ausgewählt ist, also als Bedienelement 22c in der Bedienhilfe 22 sichtbar wird und ist. Eine Verlagerung des anderen sichtbaren Punkts z₂ wird im Bedienbereich auf der Bedienhilfe 22 nicht angezeigt, da seine Technologie (die der Paretofront 301) im Funktionsfeld 80 nicht ausgewählt ist. Das zugehörige Kontrollkästchen 82 ist abgewählt. Daraus ersichtlich ist, dass der primär für die Bedienung des Planers vorgesehene Bedienbereich 2 nur eine Option ist. Eine Bedienung kann auch aus dem Patchbereich 1 heraus erfolgen, mit Blick auf die dargestellten, interaktiv verfügbaren Zielpunkte zᵢ.

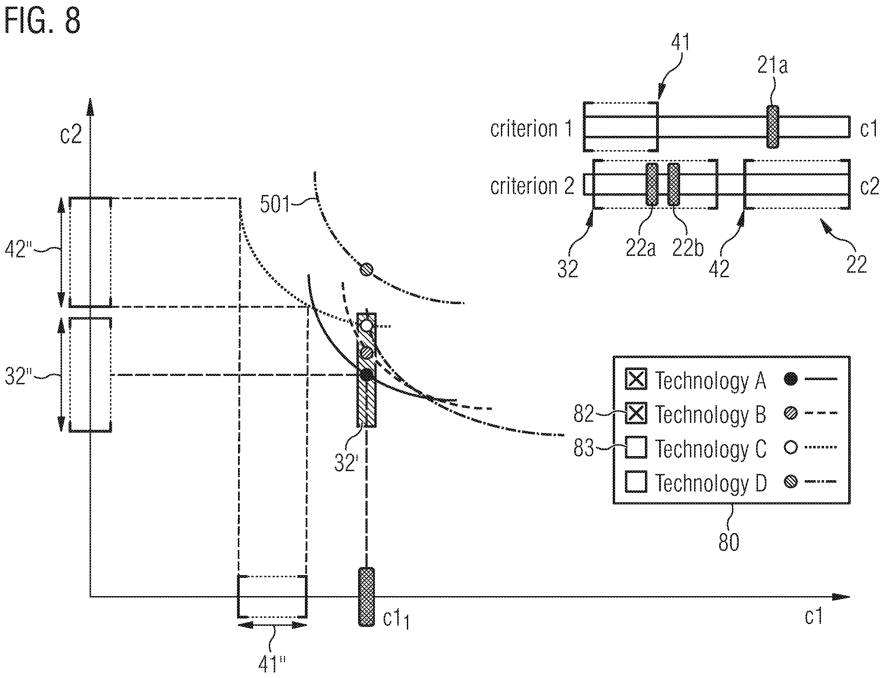

Anhand der Figur 8 soll aufgezeigt werden, dass ein Ausschluss eines Patches zum Sperren ganzer Bereiche führen kann.

Diese gesperrten Felder sind mit 41 und 42 auf den beiden Bedienelementen 21 und 22 ersichtlich. Abgebildet sind sie aus dem Bedienbereich 2 im Patchbereich 1 durch die Erstreckung 42" auf der Kriteriumsachse c2 und die Erstreckung 41" auf der Kriteriumsachse c1.

Diese beiden Ausschlussbereiche folgen dem Abwählen der Technologie C über das Kontrollkästchen 83 im Funktionsfeld 80. Da diese Technologie auf Interaktion des Planers derzeit nicht berücksichtigt werden soll, sind die zugehörigen Abschnitte 42" und 41" auf den Kriterienachsen c1 und c2 nicht verfügbar. Die Auswahlmöglichkeiten werden so beschränkt, es besteht aber weiterhin die Auswahlmöglichkeit des Unschärfeintervalls 32 für das Kriterium c2, bei konstant bleibendem Wert c1₁ auf der Achse des Kriteriums c1.

Dieses Feld 32' entspricht dem Feld 31' von Figur 7, nur ist hier die Technologie B angewählt, über das Kontrollkästchen 82, sodass auch ein anderes Bedienelement 22b im Bedienbereich 2 auf der Bedienhilfe 22 erscheint, dagegen aufgrund der Abwahl der Technologie C über das Deaktivieren des Kontrollkästchens 83 das Bedienelement 22c fehlt.

Im gezeigten Beispiel führt der Ausschuss des Patches 401 zu einer Beschränkung der Auswahlmöglichkeiten. Durch Ausschluss dieses Patches fallen die "besten Optionen" des Kriteriums c1 weg.

Die zugehörigen Felder 42" und 41" können auf dem Sichtschirm farbig markiert werden, sinnvollerweise in einer Signalfarbe, um deren Nichtverfügbarkeit im Patchbereich zu kennzeichnen, aber auch mit einer Signalfarbe in den zugehörigen Ausschlussintervallen 41 und 42 im Bedienbereich 2 zu kennzeichnen.

Wird in allen Beispielen das andere Kriterium c2 auf seiner Achse festgehalten, erstrecken sich die zuvor beschriebenen vertikalen Ähnlichkeitsfelder horizontal. Entsprechend sind auch diese Orientierungen offenbart. Die Beschreibung einer Richtung oder Orientierung ist keine Beschränkung darauf.

Figur 9 zeigt eine netzwerkorientierte Darstellung der Steuerung über einen Bus 701 und die daran angekoppelten und verwendeten digitalen Komponenten. Der Bus 701 ist im bevorzugten Beispiel einen 64 Bit Bus, über den die Komponenten miteinander kommunizieren. Er ist bidirektional. Für die Rechenkapazität und die Rechenfunktion ist die CPU 700 vorgesehen, welche sich die auf dem Display 10 dargestellten Funktionen 101, 201 usw. aus dem Speicher 750 ausliest, und sie der Anzeigeeinheit I/O 760 über den Bus 701 weiterleitet. In der Anzeigeeinheit 760 werden die Daten je nach technischen Typ des Displays 10 daten-technisch aufbereitet und sichtbar dargestellt. Dargestellt ist die Navigationsumgebung der Figur 1. Sie wird über den Funktionszeiger M bedient und er wird vom Planer über ein Mausgerät, hier im Beispiel als Trackball M' gezeigt, bewegt und aktiviert (Anwahl einer Funktion an einem Ort des Zeigens des Zeigers M). Der Trackball M' ist per Funk mit einem Empfänger 710 gekoppelt, der seine Bewegungssignale umsetzt und an den Bus 701 weiterleitet. Diese Kopplung ist nicht bidirektional.

Dem Display 10 ist das Mausgerät M' zugeordnet, bspw. als der Trackball, der die Funktionen der Zeigens und des Bedienens (das Auslösen einer Funktion) ermöglicht, dies am Ort der Anzeige des Mauszeigers M.

Mit dem Mauszeiger M werden ebenso beide Bedienhilfen 21 und 22 für die Kriterien c1 und c2 bedient.

Ein alternatives Display 781 ist das eines Tablets 780, welches mit der gleichen Darstellung der Fig. 1 versehen ist, welche auf dem touch-sensitiven Display 781 dargestellt wird. Die Bildschirmanzeige oder die Tablet-Anzeige hat einen Patchbereich 1 und den Bedienbereich 2, wie zuvor erläutert. Das Tablet 780 ist über eine WLAN-Kopplung (oder Anbindung) mit einem WLAN-Sender und Empfänger 770 an dem Bus 701 angekoppelt. Der Mauszeiger wird durch den Finger (wie dargestellt) ersetzt und durch Gesten (z.B. Swipe, Tap, double Tap) auf dem Display 781 des screen-enabled oder touch-sensitiven Tablet 780 gesteuert.

Die technologischen Geräte 100 bis 400 sind über jeweils ein Input/Output Device 731, 732, 733 und 734 an dem Bus 701 angekoppelt. Jedes dieser Input/Output Devices ist bidirektional, kann also einstellende Parameter von dem Bus 701 auf das jeweilige Gerät für seine nach der Einstellung erfolgende Benutzung vorgeben, bevorzugt hat jedes dieser Geräte 100, 200, ... einen ausreichenden Speicherplatz von zumindest 500GB, so dass es die vor-eingestellten Parameter der späteren Therapie speichern und während der Therapie eigenständig, also autark einstellen kann, besonders für fraktionierte Sitzungen der Patienten über den Planungszeitraum. Im Beispiel sendet I/O₁ die Einstellparameter an das Gerät 100 zur Abgabe von Protonen. Dieses ist dann in der Lage, die einzustellenden, eigentlich die dann bereits vorgegebenen Parameter des Protonengeräts 100 während der über Tage verteilten (fraktionierten) Therapie des Patienten P anzuwenden. Also die Dosen und Intensitäten und Richtungen an Protonenstrahlungen (in der Radiotherapie) an diesen individuellen Patienten P abzugeben.

Dieses Beispiel ist auch so steuerbar, dass die Parameter im Speicher 750 über die Dauer der fraktionierten Therapie vorgehalten werden, und nur zu den Zeiten vor der jeweiligen Therapiesitzung an das Strahlengerät 100 zu übertragen. Diese fraktionierte Therapie ist dabei eine fraktionierte Programmierung des jeweiligen Geräts 100 bis 400 (im Sinne der fraktionierten Datenübertragung jeweils des aktuell benötigten Abschnitts der Therapie von z.B. 30 Tagen). Die eigentliche Therapie führt das Gerät 100 selbsttätig, und ohne die schon zuvor erfolgte und fertige Planung durch.

In gleicher Weise werden die anderen Geräte 200, 300 und 400 programmiert, für die Therapie vorbereitet und datentechnisch konditioniert.

Es ist hier zu betonen, dass keine Therapie gleichzeitig mit der Planung erfolgt, die Therapie ist bereits fertig geplant und funktionell vorbereitet (also fertig geplant), bevor das jeweilige Gerät diese Radiotherapie am Patienten ausführt. Letztere Therapie ist nicht beansprucht.

Eine Navigation über die dargestellten Patches auf dem Display 10 kann erst vom Planer abgeschlossen werden, um sie später in das oder die zugehörigen Geräte über das jeweilige der Input/Output Devices zu übertragen.

Die Berechnung der CPU 700 führt zu einer grafischen Festlegung eines Ähnlichkeitsbereichs 31, der als Unschärfeintervall zuvor beschrieben war. Er ist auf dem Display 10 nicht dargestellt und auch nicht auf dem touch-sensitiven Display 781 des Tablets 780.

## Patentansprüche

1. **Verfahren zum Entwerfen oder Gestalten** einer Therapie als Behandlungsplan, vor einer Behandlung und mit einer interaktiven Navigation an einem Sichtgerät (10)
(a) wobei mehrere Technologien (A, B, ... Z) zumindest eines Strahlengeräts (100,200,300) zur Auswahl stehen, unter anderem zumindest eine Technologie (A) mit einem Strahlengerät (100) zur Abgabe von Protonen und/oder zumindest eine Technologie (B) mit einem Strahlengerät (200) zur Abgabe von Photonen;
(b) für eine nach dem Entwerfen oder Gestalten zu behandelnde Person (P) mit einer von dem Strahlengerät (100,200,300) therapierbare Erkrankung, wobei der entworfene oder gestaltete Plan eine Vielzahl von technischen Einstellungen definiert, welche an dem oder den Strahlengerät(en) der ausgewählten Technologie(n) einstellbar sind;
**dadurch gekennzeichnet, dass**
(c1) auf dem Sichtgerät (10) für zumindest einige der Technologien (A, B, ... Z) je eine Paretofront (101;201;301) dargestellt werden, die jeweils Patch genannt wird;
(c2) auf dem Sichtgerät (10) in einem Bedienbereich, außerhalb eines Patchbereichs, eine Anzahl von Bedienhilfen (21, 22, ...), insbesondere als Slider, dargestellt werden, von denen jede ein Kriterium (c1,c2, ...) repräsentiert, welches bei Bewegen oder Bedienen eines Selektors (21a, 22a, ...) der jeweiligen Bedienhilfe in eine Richtung verbessert oder in eine gegenläufige Richtung verschlechtert wird;
(c3) und ein jeweiliger Selektor (21a,22a) für eine interaktive Kommunikation mit einem Planer eine im aktuellen Zustand ausgewählte Lösung im Patchbereich (1) als Punkt (zᵢ) auf einer der mehreren Paretofronten (101,201,301) darstellt, wobei die ausgewählte Lösung verändert wird, entlang mehrerer Patches, die mehrere Technologien (A, B, ... Z) navigierbar abbilden, wenn der Planer den jeweiligen Selektor der jeweiligen Bedienhilfe in seiner Lage verändert;
(d) mit einem Unschärfeintervall (30,31,32), welches bei einem Zielkriterium (c1,c2, ...) definiert wird, und welches auch im interaktiven Patchbereich entsprechend repräsentiert angezeigt wird und zumindest zwei Paretofronten (201, 301) für zumindest zwei Technologien (A,B) erfasst; wodurch interaktiv durch eine Eingabe des Planers eine Gleichwertigkeit von zwei Technologien demselben Planer vermittelt wird, und die beiden dargestellten Technologien (201,301) einen gleichen Wert (c1₁) für das gleiche Zielkriterium (c1) für den jeweiligen Patch aufweisen.

2. Verfahren nach Anspruch 1, wobei auf dem Sichtgerät (10) eine resultierende Paretofront als Patch (801) dargestellt wird, die mehrere Abschnitte von mehreren dargestellten Paretofronten als Patches (101,201,401) dargestellter Technologien (A, B, ... Z) nachführt, um es dem Planer zu erleichtern, sichtbar technologie-übergreifend auf dem resultierenden Patch (801) zu navigieren, für ein Auffinden eines Behandlungsplans.

3. Verfahren nach Anspruch 1, wobei die Lage des Selektors einer Bedienhilfe linear verändert wird, oder als Drehlage verändert wird,
oder
mehr als zwei Zielkriterien, auch Planziele, durch Projektionen im Patchbereich auf dem Sichtgerät (10) dargestellt werden.

4. Verfahren nach einem der vorigen Ansprüche, wobei eines der Zielkriterien eine Behandlungszeit ist.

5. Verfahren nach einem der vorigen Ansprüche, wobei das Unschärfeintervall (30,31) durch Verbreitern des Selektors (21a) der Bedienhilfe (21) interaktiv dargestellt wird und für die interaktive Kommunikation mit dem Planer auch im Patchbereich durch ein erweitertes Feld (30',31') repräsentiert wird, erfassend zumindest zwei Paretofronten (201,301) als zumindest zwei Patches.

6. Verfahren nach einem der Ansprüche 3 bis 5, wobei die Bedienhilfe linear ausgebildet ist, oder eine Bedienhilfe mit einem gebogenen Verstellbereich ist.

7. Verfahren nach einem der vorigen Ansprüche, wobei das oder die Strahlengeräte ausgebildet sind, eine Person (P) mit einer Tumorerkrankung zu behandeln.

8. Verfahren nach einem der vorigen Ansprüche, wobei auf dem Sichtgerät (10) ein Fenster als Funktionsfeld (80) angezeigt wird, in welchem der Planer ausgewählte Technologien kennzeichnet.

9. Verfahren nach Anspruch 8, wobei über das im Bedienbereich (1) dargestellte Fenster als Funktionsfeld (80) Technologien angezeigt werden, die den Paretofronten (201,202) entsprechen, zur Auswahl oder Abwahl durch den Planer, oder die vom Planer ausgewählt werden und bei Auswahl in den Bedienhilfen (21,22) mit angezeigt oder dargestellt werden. ...

10. **Technisch einsetzbare** Benutzerschnittstelle, GUI, als grafisch-interaktives Interface zur interaktiven Bedienung von einem Planer mit einem Funktionszeiger (M) auf oder an einem Sichtgerät (10) zur Ausführung des Verfahrens nach einem der vorigen Ansprüche, wobei die technisch einsetzbare GUI
(a1) auf einem Sichtgerät (10) des Eingabe-/Ausgabegeräts für zumindest einige Technologien je eine Paretofront (101,201,301) in einem Paretofront-Diagramm dargestellt ist, und jede der Paretofronten Patch genannt wird, wobei jede der Paretofronten in Abhängigkeit von zumindest zwei Kriterien (c1,c2) in einem Patchbereich (1) des Sichtgeräts (10) dargestellt ist;
(a2) auf dem Sichtgerät (10) in einem Bedienbereich (2), außerhalb des Patchbereichs (1), eine Anzahl von Bedienhilfen (21, 22, ...) dargestellt sind, von denen jede Bedienhilfe ein Kriterium (c1,c2,...) repräsentiert, und welche Bedienhilfen jeweils einen Selektor (21a,22a, ...) aufweist, wobei bei einem Bewegen oder Bedienen eines Selektors (22a) der jeweiligen Bedienhilfe in eine Richtung ein entsprechendes Kriterium verbesserbar oder in eine gegenläufige Richtung verschlechterungsfähig ist;
(a3) und jede Bedienhilfe (21, 22, ...) mit Hilfe des zugehörigen Selektors (21a,22a, ...) - für eine interaktive Kommunikation mit dem Planer - eine im aktuellen Zustand ausgewählte Lösung in dem Patchbereich (1) als Punkt (zᵢ) auf einer der mehreren Paretofronten (101,201,301) darstellt, wobei die ausgewählte Lösung, entlang mehrerer Patches veränderbar ist;
(b) ein Unschärfeintervall (30,31,32) bestimmbar ist, das bei einem Zielkriterium (c1,c2,...) definiert wird, welches auch im interaktiven Patchbereich durch einen optisch hervorgehobenen Streifen entsprechend repräsentiert angezeigt ist und zumindest zwei Paretofronten (201, 301) als zumindest zwei Behandlungstechnologien mit seiner Erstreckung (30";31";32") erfasst; wodurch interaktiv durch eine Eingabe des Planers eine Gleichwertigkeit von zwei Technologien demselben Planer vom Patchbereich (1) so vermittelbar ist, dass die beiden dargestellten Behandlungstechnologien einen gleichen Wert (c1₁) des gleichen Zielkriteriums (c1) im jeweiligen Patch aufweisen.

11. GUI nach dem Anspruch 10, wobei das Unschärfeintervall (30,31,32) im interaktiven Patchbereich vertikal oder horizontal orientiert repräsentiert angezeigt ist.

12. GUI nach einem der vorigen Ansprüche 10 oder 11, wobei das Unschärfeintervall (30,31,32) kleiner als 10%-Punkte ist.

13. GUI nach einem der vorigen Ansprüche 10 bis 12, wobei die Selektoren (21a, 22a) funktionell mit den zugehörigen Punkten (zᵢ) auf einer der mehreren Paretofronten gekoppelt sind.

14. GUI nach einem der vorigen Ansprüche 10 bis 13, wobei über ein im Bedienbereich (1) dargestelltes Feld (80) Technologien angezeigt sind, die den Paretofronten (201,202) entsprechen, zur Auswahl oder Abwahl durch den Planer, wobei sie bei Auswahl in den Bedienhilfen (21,22) mit angezeigt oder dargestellt sind.

15. **Verwendung eines Funktionszeigers (M)** in einer grafischen Benutzerschnittstelle nach Anspruch 10 zum Entwerfen oder Gestalten einer Therapie als Behandlungsplan, im Rahmen einer interaktiven Navigation eines Planers als Benutzer an einem Sichtgerät (10), wobei
(a1) auf dem Sichtgerät (10) für zumindest einige Technologien (A, B, ... Z) je eine Paretofront (101;201;301) zusammenhängend dargestellt sind, die jeweils Patch genannt wird;
(a2) in einem Bedienbereich auf dem Sichtgerät (10) eine Anzahl von Bedienhilfen (21, 22, ...) dargestellt sind, von denen jede ein Kriterium (c1,c2, ...) repräsentiert, welches ein Bewegen oder Bedienen eines Selektors (21a, 22a, ...) der jeweiligen Bedienhilfe erlaubt;
(a3) und der jeweiliger Selektor (21a,22a) für eine interaktive Kommunikation mit dem Planer eine im aktuellen Zustand ausgewählte Lösung im Patchbereich (1) als Punkt (zᵢ) auf einer der mehreren Paretofronten (101,201,301) hervorgehoben darstellt, und eine ausgewählte Lösung vom Funktionszeiger (M) verändert wird, entlang mehrerer Patches;
(b) mit einem Unschärfeintervall (30,31,32), welches bei einem Zielkriterium (c1,c2, ...) definiert wird, und welches auch im interaktiven Patchbereich entsprechend repräsentiert angezeigt wird und zumindest zwei Paretofronten (201, 301) für zumindest zwei Technologien (A,B) erfasst; wodurch interaktiv eine Gleichwertigkeit von zwei Technologien dem Planer vermittelt wird.

16. Verwendung nach Anspruch 15, wobei das Unschärfeintervall (30,31,32) im interaktiven Patchbereich vertikal oder horizontal orientiert repräsentiert angezeigt ist.

17. Verwendung nach einem der Ansprüche 15 oder 16, wobei das Unschärfeintervall (30,31,32) kleiner als 10%-Punkte ist.

18. Verwendung nach einem der Ansprüche 15 bis 17, wobei die Selektoren (21a, 22a) funktionell mit den zugehörigen Punkten (zᵢ) auf einer der mehreren Paretofronten gekoppelt sind.

19. Verwendung nach einem der Ansprüche 15 bis 18, wobei über ein im Bedienbereich (1) dargestelltes Funktionsfeld (80) Technologien angezeigt sind, die den Paretofronten (201,202) entsprechen, zur Auswahl oder Abwahl durch den Planer, wobei sie bei Auswahl in den Bedienhilfen (21,22) mit angezeigt oder dargestellt sind.

## Claims

1. **Method for designing or devising** a therapy as a treatment plan, prior to treatment and with interactive navigation on a display device (10)
(a) wherein a plurality of technologies (A, B, ... Z) of at least one radiation device (100, 200, 300) are available for selection, including at least one technology (A) with a radiation device (100) for delivering protons and/or at least one technology (B) with a radiation device (200) for delivering photons;
(b) for a person (P) to be treated according to the plan, who has a condition treatable by the radiation device (100, 200, 300), wherein the plan defines a plurality of technical settings which can be adjusted on the radiation device(s) of the selected technology(ies);
**characterised in that**
(c1) on the display device (10), a Pareto front (101; 201; 301) is displayed for at least some of the technologies (A, B, ... Z), each of which is referred to as a patch;
(c2) on the display device (10), within an operating area outside a patch area, a number of operating aids (21, 22, ...), in particular in the form of sliders, are displayed, each of which represents a criterion (c1, c2, ...) which, when a selector (21a, 22a, ...) of the respective control aid in one direction is improved or, in the opposite direction, is worsened;
(c3) and a respective selector (21a, 22a) for interactive communication with a planner displays a solution selected in the current state within the patch area (1) as a point (zᵢ ) on one of the multiple Pareto fronts (101, 201, 301), whereby the selected solution is modified, along multiple patches, which can be navigated across multiple technologies (A, B, ... Z), when the planner changes the position of the respective selector of the respective user aid;
(d) with a uncertainty interval (30, 31, 32), which is defined for a target criterion (c1, c2, ...) and which is also displayed in the interactive patch area in a corresponding manner and encompasses at least two Pareto fronts (201, 301) for at least two technologies (A, B); whereby, interactively through an input by the planner, an equivalence of two technologies is conveyed to the same planner, and the two technologies (201, 301) displayed in the have the same value (c1₁ ) for the same objective criterion (c1) for the respective patch.

2. Method according to claim 1, wherein a resulting Pareto front is displayed on the display device (10) as a patch (801), which tracks several sections of several displayed Pareto fronts as patches (101, 201, 401) of displayed technologies (A, B, ... Z) in order to make it easier for the planner to navigate visibly across technologies on the resulting patch (801) for the purpose of finding a treatment plan.

3. Method according to claim 1, wherein
the position of the selector of an operating aid is changed linearly, or is changed as a rotational position,
or
more than two target criteria, including planning targets, are displayed by projections in the patch area on the display device (10).

4. Method according to one of the preceding claims, wherein one of the target criteria is a treatment time.

5. Method according to one of the preceding claims, wherein the blurring interval (30, 31) is interactively displayed by widening the selector (21a) of the user interface (21) and, for interactive communication with the planner, is also represented in the patch area by an extended field (30', 31'), encompassing at least two Pareto fronts (201, 301) as at least two patches.

6. Method according to any one of claims 3 to 5, wherein the control aid is linear in shape, or is a control aid with a curved adjustment range.

7. Method according to one of the preceding claims, wherein the radiation device or devices are configured to treat a person (P) with a tumour.

8. Method according to any of the preceding claims, wherein a window is displayed on the visualisation device (10) as a functional field (80) in which the planner marks selected technologies.

9. Method according to claim 8, wherein technologies corresponding to the Pareto fronts (201, 202) are displayed via the window shown in the operating area (1) as a function field (80) for selection or deselection by the planner, or which are selected by the planner and, upon selection, are also displayed or presented in the operating aids (21, 22). ...

10. **Technically implementable user interface, GUI,** as a graphical-interactive interface for interactive operation by a planner with a function pointer (M) on or at a display device (10) for carrying out the method according to one of the preceding claims, wherein the technically implementable GUI
(a1) is displayed on a display device (10) of the input/output device, for at least some technologies, a Pareto front (101, 201, 301) in a Pareto front diagram, and each of the Pareto fronts is referred to as a patch, wherein each of the Pareto fronts is displayed in a patch area (1) of the display device (10) depending on at least two criteria (c1, c2) in a patch area (1) of the display device (10);
(a2) a number of operating aids (21, 22, ...) are displayed on the display device (10) in an operating area (2), outside the patch area (1), each of which operating aids represents a criterion (c1, c2, ...), and which operating aids each comprise a selector (21a, 22a, ...), whereby moving or operating a selector (22a) of the respective control aid in one direction improves a corresponding criterion, or moving it in the opposite direction worsens it;
(a3) and each control element (21, 22, ...) uses the associated selector (21a, 22a, ...) - for interactive communication with the planner - represents a solution selected in the current state within the patch area (1) as a point (zᵢ ) on one of the multiple Pareto fronts (101, 201, 301), wherein the selected solution can be modified along multiple patches;
(b) an uncertainty interval (30, 31, 32) can be determined, which is defined for a target criterion (c1, c2, ...) , which is also displayed in the interactive patch area by means of a visually highlighted strip, and which encompasses at least two Pareto fronts (201, 301) as at least two treatment technologies with its extent (30"; 31"; 32"); whereby, interactively via an input from the planner, an equivalence of two technologies can be conveyed to the same planner from the patch area (1) in such a way that the two depicted treatment technologies have the same value (c1₁) of the same target criterion (c1) in the respective patch.

11. GUI according to claim 10, wherein the uncertainty interval (30, 31, 32) is displayed in the interactive patch area in a vertically or horizontally oriented manner.

12. GUI according to any of the preceding claims 10 or 11, wherein the blur interval (30, 31, 32) is less than 10 percentage points.

13. GUI according to any one of the preceding claims 10 to 12, wherein the selectors (21a, 22a) are functionally coupled to the associated points (zᵢ ) on one of the plurality of Pareto fronts.

14. GUI according to any one of the preceding claims 10 to 13, wherein technologies corresponding to the Pareto fronts (201, 202) are displayed via a field (80) shown in the user interface (1) for selection or deselection by the planner, and wherein, upon selection, they are also displayed or shown in the user aids (21, 22).

15. **Use of a function pointer (M)** in a graphical user interface according to claim 10 for designing or creating a therapy as a treatment plan, within the framework of interactive navigation by a planner as a user at a display device (10), wherein
(a1) on the display device (10), for at least some technologies (A, B, ... Z), a Pareto front (101; 201; 301) is displayed contiguously for each, which is referred to as a patch;
(a2) a number of control aids (21, 22, ...) are displayed in a control area on the display device (10), each of which represents a criterion (c1, c2, ...) that allows a selector (21a, 22a, ...) of the respective control aid to be moved or operated;
(a3) and the respective selector (21a, 22a) for interactive communication with the planner highlights a solution selected in the current state within the patch area (1) as a point (zᵢ ) on one of the multiple Pareto fronts (101, 201, 301), and a selected solution is modified by the function pointer (M) along multiple patches;
(b) with a fuzzy interval (30, 31, 32), which is defined for a target criterion (c1, c2, ...) and which is also displayed in the interactive patch area in a corresponding manner and encompasses at least two Pareto fronts (201, 301) for at least two technologies (A, B); thereby interactively conveying an equivalence of two technologies to the planner.

16. Use according to claim 15, wherein the uncertainty interval (30, 31, 32) is displayed in the interactive patch area in a vertically or horizontally oriented manner.

17. Use according to one of claims 15 or 16, wherein the uncertainty interval (30, 31, 32) is less than 10 percentage points.

18. Use according to any one of claims 15 to 17, wherein the selectors (21a, 22a) are functionally coupled to the associated points (zᵢ ) on one of the plurality of Pareto fronts.

19. Use according to one of claims 15 to 18, wherein technologies corresponding to the Pareto fronts (201, 202) are displayed via a function field (80) shown in the operating area (1) for selection or deselection by the planner, wherein, upon selection, they are also displayed or shown in the operating aids (21, 22).

## Revendications

1. **Procédé de conception ou d'élaboration** d'une thérapie sous forme de plan de traitement, avant un traitement et avec une navigation interactive sur un dispositif d'affichage (10)
(a) dans lequel plusieurs technologies (A, B, ... Z) d'au moins un appareil à rayonnement (100, 200, 300) sont disponibles, notamment au moins une technologie (A) avec un appareil à rayonnement (100) pour l'émission de protons et/ou au moins une technologie (B) avec un appareil à rayonnement (200) pour l'émission de photons ;
(b) pour une personne (P) à traiter selon le projet ou la conception, atteinte d'une maladie pouvant être traitée par le dispositif de rayonnement (100, 200, 300), le plan projeté ou conçu définissant une pluralité de paramètres techniques qui peuvent être réglés sur le ou les dispositifs de rayonnement de la ou des technologies sélectionnées ;
**caractérisé en ce que**
(c1) sur le dispositif d'affichage (10), pour au moins certaines des technologies (A, B, ... Z), est représenté un front de Pareto (101 ; 201 ; 301), appelé respectivement «patch» ;
(c2) sur le dispositif d'affichage (10), dans une zone de commande située en dehors d'une zone de patch, sont représentés un certain nombre d'aides à la commande (21, 22, ...), notamment sous forme de curseurs, dont chacune représente un critère (c1, c2, ...) qui, lors du déplacement ou de l'actionnement d'un sélecteur (21a, 22a, ...) de l'aide à la commande correspondante est amélioré dans un sens ou détérioré dans le sens opposé ;
(c3) et un sélecteur respectif (21a, 22a) représente, pour une communication interactive avec un planificateur, une solution sélectionnée dans l'état actuel dans la zone de patch (1) sous la forme d'un point (zᵢ ) sur l'un des multiples fronts de Pareto (101, 201, 301), la solution sélectionnée pouvant être modifiée le long de multiples patches, sur lesquels plusieurs technologies (A, B, ... Z) de manière navigable lorsque le planificateur modifie la position du sélecteur correspondant de l'aide à l'utilisation correspondante ;
(d) avec un intervalle de flou (30, 31, 32) qui est défini pour un critère cible (c1, c2, ...) et qui est également affiché de manière représentative dans la zone de patch interactive et englobe au moins deux fronts de Pareto (201, 301) pour au moins deux technologies (A, B) ; ce qui permet de communiquer de manière interactive, par une saisie du planificateur, une équivalence entre deux technologies au même planificateur, et les deux technologies (201, 301) représentées de manière e présentent une valeur identique (c1₁ ) pour le même critère objectif (c1) pour le patch concerné.

2. Procédé selon la revendication 1, dans lequel un front de Pareto résultant est représenté sur le dispositif d'affichage (10) sous la forme d'un patch (801), qui suit plusieurs sections de plusieurs fronts de Pareto représentés sous forme de patches (101, 201, 401) de technologies représentées (A, B, ... Z) afin de faciliter la navigation visuelle du planificateur sur l'ensemble des technologies représentées sur le patch résultant (801), en vue de la recherche d'un plan de traitement.

3. Procédé selon la revendication 1, dans lequel
la position du sélecteur d'une aide à l'utilisation est modifiée de manière linéaire ou en tant que position angulaire,
ou
plus de deux critères cibles, y compris des objectifs de planification, sont représentés par des projections dans la zone du patch sur l'écran (10).

4. Procédé selon l'une des revendications précédentes, dans lequel l'un des critères cibles est une durée de traitement.

5. Procédé selon l'une des revendications précédentes, dans lequel l'intervalle de flou (30, 31) est représenté de manière interactive par l'élargissement du sélecteur (21a) de l'aide à la commande (21) et est également représenté, pour la communication interactive avec le planificateur, dans la zone de patch par un champ élargi (30', 31'), englobant au moins deux fronts de Pareto (201, 301) sous la forme d'au moins deux patchs.

6. Procédé selon l'une des revendications 3 à 5, dans lequel l'aide à la commande est de forme linéaire, ou est une aide à la commande avec une zone de réglage courbe.

7. Procédé selon l'une des revendications précédentes, dans lequel le ou les appareils de radiothérapie sont conçus pour traiter une personne (P) atteinte d'une tumeur.

8. Procédé selon l'une des revendications précédentes, dans lequel une fenêtre est affichée sur l'écran (10) en tant que champ fonctionnel (80), dans lequel le planificateur marque les technologies sélectionnées.

9. Procédé selon la revendication 8, dans lequel, via la fenêtre affichée dans la zone de commande (1) en tant que champ fonctionnel (80), des technologies correspondant aux fronts de Pareto (201, 202) sont affichées pour être sélectionnées ou désélectionnées par le planificateur, ou sont sélectionnées par le planificateur et, une fois sélectionnées, sont également affichées ou représentées dans les aides à la commande (21, 22).

10. **Interface utilisateur techniquement utilisable, GUI,** servant d'interface graphique interactive pour la commande interactive par un planificateur à l'aide d'un pointeur de fonction (M) sur ou à un dispositif d'affichage (10) pour l'exécution du procédé selon l'une des revendications précédentes, l'interface utilisateur techniquement utilisable (GUI)
(a1) est représentée sur un dispositif d'affichage (10) du dispositif d'entrée/sortie, pour au moins certaines technologies, par un front de Pareto (101, 201, 301) dans un diagramme de fronts de Pareto, et chacun des fronts de Pareto est appelé « patch », chacun des fronts de Pareto étant représenté en fonction d'au moins deux critères (c1, c2) dans une zone de patch (1) de l'écran (10) ;
(a2) sur l'écran (10), dans une zone de commande (2) située en dehors de la zone de patch (1), sont représentées plusieurs aides à la commande (21, 22, ...), dont chacune représente un critère (c1, c2, ...), et lesquelles aides à la commande comportent chacune un sélecteur (21a, 22a, ...), de sorte que, lorsqu'un sélecteur (22a) de l'aide à la commande correspondante est déplacé ou actionné dans un sens, un critère correspondant peut être amélioré, ou dans le sens opposé, détérioré ;
(a3) et chaque aide à la commande (21, 22, ...) permet, à l'aide du sélecteur associé (21a, 22a, ...) - pour une communication interactive avec le planificateur - représente une solution sélectionnée dans l'état actuel dans la zone de patch (1) sous la forme d'un point (zᵢ ) sur l'un des multiples fronts de Pareto (101, 201, 301), la solution sélectionnée pouvant être modifiée le long de plusieurs patches ;
(b) un intervalle de flou (30, 31, 32) peut être déterminé, qui est défini pour un critère cible (c1, c2, ...) , lequel est également représenté de manière correspondante dans la zone de patch interactive par une bande mise en évidence visuellement et englobe au moins deux fronts de Pareto (201, 301) en tant qu'au moins deux technologies de traitement avec son étendue (30" ; 31" ; 32") ; ce qui permet, de manière interactive, par une saisie du planificateur, de communiquer à ce même planificateur une équivalence entre deux technologies à partir de la zone de patch (1), de telle sorte que les deux technologies de traitement représentées présentent une valeur identique (c1₁) du même critère cible (c1) dans le patch respectif.

11. Interface graphique selon la revendication 10, dans laquelle l'intervalle de flou (30, 31, 32) est représenté et affiché de manière orientée verticalement ou horizontalement dans la zone de patch interactive.

12. Interface graphique selon l'une des revendications 10 ou 11 précédentes, dans laquelle l'intervalle de flou (30, 31, 32) est inférieur à 10 points de pourcentage.

13. Interface utilisateur graphique selon l'une des revendications 10 à 12 précédentes, dans laquelle les sélecteurs (21a, 22a) sont couplés fonctionnellement aux points associés (zᵢ ) sur l'un des multiples fronts de Pareto.

14. Interface graphique utilisateur selon l'une des revendications précédentes 10 à 13, dans laquelle des technologies correspondant aux fronts de Pareto (201, 202) sont affichées via un champ (80) représenté dans la zone de commande (1) afin d'être sélectionnées ou désélectionnées par le planificateur, et sont affichées ou représentées dans les aides à la commande (21, 22) lorsqu'elles sont sélectionnées.

15. **Utilisation d'un pointeur de fonction (M)** dans une interface utilisateur graphique selon la revendication 10 pour concevoir ou élaborer une thérapie sous forme de plan de traitement, dans le cadre d'une navigation interactive d'un planificateur en tant qu'utilisateur sur un dispositif d'affichage (10), dans laquelle
(a1) sur le dispositif d'affichage (10), pour au moins certaines technologies (A, B, ... Z), un front de Pareto (101 ; 201 ; 301) est représenté de manière cohérente pour chacune d'entre elles, lequel est appelé « patch » ;
(a2) dans une zone de commande sur le dispositif d'affichage (10), un certain nombre d'aides à la commande (21, 22, ...) sont représentées, dont chacune représente un critère (c1, c2, ...) qui permet de déplacer ou d'actionner un sélecteur (21a, 22a, ...) de l'aide à la commande correspondante ;
(a3) et le sélecteur correspondant (21a, 22a) affiche, pour une communication interactive avec le planificateur, une solution sélectionnée dans l'état actuel dans la zone de patch (1) sous la forme d'un point (zᵢ ) sur l'un des multiples fronts de Pareto (101, 201, 301), et une solution sélectionnée est modifiée par le pointeur de fonction (M) le long de plusieurs patchs ;
(b) avec un intervalle de flou (30, 31, 32) qui est défini pour un critère cible (c1, c2, ...) et qui est également affiché de manière représentative dans la zone de patch interactive et englobe au moins deux fronts de Pareto (201, 301) pour au moins deux technologies (A, B) ; ce qui permet de communiquer de manière interactive une équivalence entre deux technologies au planificateur.

16. Utilisation selon la revendication 15, dans laquelle l'intervalle de flou (30, 31, 32) est représenté et affiché dans la zone interactive de manière orientée verticalement ou horizontalement.

17. Utilisation selon l'une des revendications 15 ou 16, dans laquelle l'intervalle de flou (30, 31, 32) est inférieur à 10 points de pourcentage.

18. Utilisation selon l'une des revendications 15 à 17, dans laquelle les sélecteurs (21a, 22a) sont couplés fonctionnellement aux points associés (zᵢ ) sur l'un des multiples fronts de Pareto.

19. Utilisation selon l'une des revendications 15 à 18, dans laquelle des technologies correspondant aux fronts de Pareto (201, 202) sont affichées via un champ de fonctions (80) représenté dans la zone de commande (1), afin d'être sélectionnées ou désélectionnées par le planificateur, et sont affichées ou représentées dans les aides à la commande (21, 22) lorsqu'elles sont sélectionnées.
